(19) Europäisches Patentamt
European Patent Office
Office européen des brevets

(11) EP 3 539 540 B1

(12) **EUROPEAN PATENT SPECIFICATION**

(45) Date of publication and mention of the grant of the patent:
**18.12.2024 Bulletin 2024/51**

(51) International Patent Classification (IPC):
***A61K 31/341*** *(2006.01)* ***A61K 31/00*** *(2006.01)*
***A61K 38/28*** *(2006.01)* ***A61P 3/10*** *(2006.01)*

(21) Application number: **19163940.0**

(22) Date of filing: **07.11.2011**

(54) **SGLT2 INHIBITOR 1-CHLORO-4-(BETA-D-GLUCOPYRANOS-1-YL)-2-[4-((S)-TETRAHYDROFURAN-3-YLOXY)-BENZYL]-BENZENE IN COMBINATION WITH INSULIN FOR USE IN A METHOD FOR REDUCING THE RISK OF HYPOGLYCEMIA**

SGLT2 INHIBITOR 1-CHLORO-4-(BETA-D-GLUCOPYRANOS-1-YL)-2-[4-((S)-TETRAHYDROFURAN-3-YLOXY)-BENZYL]-BENZENE IN KOMBINATION MIT INSULIN ZUR VERWENDUNG IN EINER METHODE ZUR REDUKTION DES RISIKOS EINER HYPOGLYKÄMIE

SGLT2 INHIBITOR 1-CHLORO-4-(BETA-D-GLUCOPYRANOS-1-YL)-2-[4-((S)-TETRAHYDROFURAN-3-YLOXY)-BENZYL]-BENZENE EN COMBINAISON AVEC INSULINE POUR LEUR UTILISATION DANS UN PROCÉDÉ DE LA RÉDUCTION DU RISQUE DE LA HYPOGLYCÉMIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.11.2010 EP 10190303**
**17.01.2011 EP 11151059**

(43) Date of publication of application:
**18.09.2019 Bulletin 2019/38**

(62) Document number(s) of the earlier application(s) in accordance with Art. 76 EPC:
**11778916.4 / 2 637 648**

(27) Previously filed application:
**07.11.2011 EP PCT/EP2011/069532**

(73) Proprietor: **Boehringer Ingelheim International GmbH**
**55216 Ingelheim am Rhein (DE)**

(72) Inventors:
- **GREMPLER, Rolf**
**55216 INGELHEIM AM RHEIN (DE)**
- **JOHANSEN, Odd-Erik**
**55216 INGELHEIM AM RHEIN (DE)**
- **KLEIN, Thomas**
**55216 INGELHEIM AM RHEIN (DE)**
- **LUIPPOLD, Gerd**
**55216 INGELHEIM AM RHEIN (DE)**
- **MARK, Michael**
**55216 INGELHEIM AM RHEIN (DE)**

(74) Representative: **Lutze, Oliver et al**
**Boehringer Ingelheim International GmbH**
**Binger Straße 173**
**55216 Ingelheim am Rhein (DE)**

(56) References cited:

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 539 540 B1

## Description

### Technical Field of the Invention

The invention relates to a pharmaceutical composition comprising an SGLT2-inhibitor and an insulin as described hereinafter which is suitable in the treatment, prevention or reducing the risk of hypoglycemia.

Furthermore the invention relates to methods for treating, preventing or reducing the risk of hypoglycemia in patients in need thereof characterized in that an SGLT2 inhibitor and an insulin is administered in combination or alternation.

The invention also relates to a pharmaceutical composition according to this invention for use in a method as described hereinbefore and hereinafter.

### Background of the Invention

Type 1 diabetes mellitus (Type 1 diabetes), also called insulin dependent diabetes mellitus or juvenile diabetes, is a form of diabetes mellitus that results from autoimmune destruction of insulin-producing beta cells of the pancreas. The subsequent lack of insulin leads to increased blood glucose concentrations and increased urinary glucose excretion. The classical symptoms are polyuria, polydipsia, polyphagia, and weight loss. Type 1 diabetes may be fatal unless treated with insulin. Complications may be associated with both hypoglycemic and hyperglycemic states. Serious hypoglycemia may lead to seizures or episodes of unconsciousness requireing emergency treatment. Uncontrolled hyperglycemia and insufficient insulin may lead to severe ketoacidosis which could be fatal. Hyperglycaemia per se might also in the short term lead to tiredness and visual disturbances and can also result in long term damage to organs such as eyes, kidneys and joints. Subcutaneous injections are the most common method of administering insulin although inhaled insulin, as well as oral formulations, are being tested in clinical trials. Rapid acting insulin analogs have been developed which are more readily absorbed from the injection site and therefore act faster than rapid human insulin injected subcutaneously, intended to supply the bolus level of insulin needed after a meal. Other insulin analogs - so called long acting insulins (for example glargine insulin, detemir insulin) - are available which are released slowly over a period of time, for example 8 to 24 hours, intended to supply the basal level of insulin for the day.

Type 2 diabetes is an increasingly prevalent disease that due to a high frequency of complications associated with a reduction in life expectancy. Because of diabetes-associated microvascular complications, type 2 diabetes is currently the most frequent cause of adult-onset loss of vision, renal failure, and amputations in the industrialized world. In addition, the presence of type 2 diabetes is associated with a two to five fold increase in cardiovascular disease risk.

After long duration of disease, most patients with type 2 diabetes will eventually fail on oral therapy and become insulin dependent with the necessity for daily injections and multiple daily glucose measurements.

The UKPDS (United Kingdom Prospective Diabetes Study) demonstrated that intensive treatment with metformin, sulfonylureas or insulin resulted in only a limited improvement of glycemic control (difference in HbA1c -0.9%) as compared to conventional treatment. In addition, even in patients within the intensive treatment arm glycemic control deteriorated significantly over time and this was attributed to deterioration of beta-cell function. Of importance however, despite this deterioration of beta-cell function, was that intensive glycaemic treatment was associated with microvascular benefits in the short term (6 years) and macro-mascular benefits in the long term (15 years). Similar phenomenon has also been demonstrated in patients with type 1 diabetes mellitus, e.g. in the diabetes control and complications trial (DCCT) where a difference in the median HbA1c (-1.9%) between the conventional therapy and intensive therapy group during 6.5 years of study, led to significant relative risk reductions for microvascular complications whereas macrovascular benefits was noted 11 years after the DCCT, e.g. as reported in the EDIC (Epidemiology of Diabetes Interventions and Complications) study, where a relative HbA1c reduction by 10% in one patient compared to another was associated with a hazard ratio of 0.80 for cardiovascular complications. Despite such convincing long term effects of glycaemic management many patients with type 2 diabetes or type 1 diabetes remain inadequately treated, partly because of limitations in long term efficacy, tolerability and dosing inconvenience of existing antihyperglycemic therapies.

Oral antidiabetic drugs conventionally used in therapy (such as e.g. first- or second-line, and/or mono- or (initial or add-on) combination therapy) include, without being restricted thereto, metformin, sulphonylureas, thiazolidinediones, glinides and $\alpha$-glucosidase inhibitors.

The high incidence of therapeutic failure might be a major contributor to the high rate of long-term hyperglycemia-associated complications or chronic damages (including micro- and macrovascular complications such as e.g. diabetic nephrophathy, retinopathy or neuropathy, or cardiovascular complications) in patients with type 2 diabetes or type 1 diabetes.

Therefore, there is an unmet medical need for methods, medicaments and pharmaceutical compositions with a good efficacy with regard to glycemic control, with regard to disease-modifying properties and with regard to reduction of cardiovascular morbidity and mortality while at the same time showing an improved safety profile.

SGLT2 inhibitors inhibitors represent a novel class of agents that are being developed for the treatment or improvement in glycemic control in patients with type 2 diabetes. Glucopyranosyl-substituted benzene derivative are described in the prior art as SGLT2 inhibitors, for example in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940. The glucopyranosyl-substituted benzene derivatives are proposed as inducers of urinary sugar excretion and as medicaments in the treatment of diabetes.

Renal filtration and reuptake of glucose contributes, among other mechanisms, to the steady state plasma glucose concentration and can therefore serve as an antidiabetic target. Reuptake of filtered glucose across epithelial cells of the kidney proceeds via sodium-dependent glucose cotransporters (SGLTs) located in the brush-border membranes in the tubuli along the sodium gradient. There are at least 3 SGLT isoforms that differ in their expression pattern as well as in their physico-chemical properties. SGLT2 is exclusively expressed in the kidney, whereas SGLT1 is expressed additionally in other tissues like intestine, colon, skeletal and cardiac muscle. SGLT3 has been found to be a glucose sensor in interstitial cells of the intestine without any transport function. Potentially, other related, but not yet characterized genes, may contribute further to renal glucose reuptake. Under normoglycemia, glucose is completely reabsorbed by SGLTs in the kidney, whereas the reuptake capacity of the kidney is saturated at glucose concentrations higher than 10mM, resulting in glucosuria (hence the notion "diabetes mellitus"). This threshold concentration can be decreased by SGLT2-inhibition. It has been shown in experiments with the SGLT inhibitor phlorizin that SGLT-inhibition will partially inhibit the reuptake of glucose from the glomerular filtrate into the blood leading to a decrease in blood glucose concentrations and to glucosuria.

## Aim of the present invention

The aim of the present invention is to provide a pharmaceutical composition and method for preventing, slowing progression of, delaying or treating a metabolic disorder, in particular of diabetes mellitus and complications of diabetes mellitus.

Another aim of the present invention is to provide a pharmaceutical composition and method for treating patients with type 1 diabetes mellitus.

A further aim of the present invention is to provide a pharmaceutical composition and method for improving glycemic control in a patient in need thereof, in particular in patients with type 1 or type 2 diabetes mellitus.

Another aim of the present invention is to provide a pharmaceutical composition and method for improving glycemic control in a patient.

Another aim of the present invention is to provide a pharmaceutical composition and method for prolonging the duration of efficacy of an insulin administered to a patient.

Another aim of the present invention is to provide a pharmaceutical composition and method for reducing the required insulin dose in a patient.

Another aim of the present invention is to provide a pharmaceutical composition and method for preventing, slowing or delaying progression from impaired glucose tolerance (IGT), impaired fasting blood glucose (IFG), insulin resistance and/or metabolic syndrome to type 2 diabetes mellitus.

Yet another aim of the present invention is to provide a pharmaceutical composition and method for preventing, slowing progression of, delaying or treating of a condition or disorder from the group consisting of complications of diabetes mellitus, in particular type 1 or type 2 diabetes mellitus.

A further aim of the present invention is to provide a pharmaceutical composition and method for reducing the weight or preventing or attenuating an increase of the weight in a patient in need thereof.

Another aim of the present invention is to provide a new pharmaceutical composition with a high efficacy for the treatment of metabolic disorders, in particular of diabetes mellitus, impaired glucose tolerance (IGT), impaired fasting blood glucose (IFG), and/or hyperglycemia, which has good to very good pharmacological and/or pharmacokinetic and/or physicochemical properties.

Further aims of the present invention become apparent to the one skilled in the art by description hereinbefore and in the following and by the examples.

## Summary of the Invention

Embodiments and aspects hereinbefore and hereinafter apply as embodiments and aspects according to the invention as long as they are covered by the appended claims. Embodiments and aspects hereinbefore and hereinafter not covered by the appended claims apply as embodiments and aspects according to this disclosure. Any references in the description to methods of treatment refer to the particular compound or pharmaceutical composition for use in a method of treatment of the patient.

Within the scope of the present invention it has now surprisingly been found that a combination of a SGLT2

inhibitor and an insulin leads to a higher blood glucose lowering compared with a treatment using the insulin or the SGLT2 inhibitor alone. Thus in order to achieve a certain level of baseline blood glucose the dose of the insulin may be reduced by using a combination of a SGLT2 inhibitor and an insulin. Furthermore it has surprisingly been found that an administration of a SGLT2 inhibitor in a time period after the administration of an insulin prolonges the lowering of the blood glucose compared with an administration of the insulin alone.

Therefore a combination of a SGLT2 inhibitor and an insulin can advantageously be used for preventing, slowing progression of, delaying or treating a metabolic disorder, in particular for improving glycemic control in patients. This opens up new therapeutic possibilities in the treatment and prevention of type 1 diabetes mellitus, type 2 diabetes mellitus, complications of diabetes mellitus and of neighboring disease states.

In a first aspect of the present invention there is provided the SGLT2 inhibitor 1-chloro-4-(β-D-glucopyranos-1-yl)-2-[4-((S)-tetrahydrofuran-3-yloxy)-benzyl]-benzene for use in a method for treating, preventing or reducing the risk of hypoglycemia in a patient in need thereof characterized in that the SGLT2 inhibitor is administered with an insulin.

Therefore, in another aspect the present invention provides a pharmaceutical composition comprising

(a) the SGLT2 inhibitor, and
(b) an insulin.

According to another aspect of the disclosure, there is provided a method for treating diabetes mellitus in a patient characterized in that the SGLT2 inhibitor and an insulin are administered, for example in combination or alternation, to the patient.

According to another aspect of the disclosure, there is provided a method for treating diabetes mellitus in a patient where treatment with insulin is requried characterized in that the SGLT2 inhibitor and an insulin are administered, for example in combination or alternation, to the patient.

According to another aspect of the disclosure, there is provided a method for treating type 1 diabetes mellitus in a patient characterized in that the SGLT2 inhibitor and an insulin are administered, for example in combination or alternation, to the patient.

According to another aspect of the invention, there is provided a method for treating, preventing or reducing the risk of hypoglycemia in a patient characterized in that the SGLT2 inhibitor and an insulin are administered, for example in combination or alternation, to the patient.

According to another aspect of the disclosure, there is provided a method for preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis, in a patient in need thereof characterized in that the SGLT2 inhibitor and an insulin are administered, for example in combination or alternation, to the patient. In particular one or more aspects of diabetic nephropathy such as hyperperfusion, proteinuria and albuminuria may be treated, their progression slowed or their onset delayed or prevented. The term "tissue ischaemia" particularly comprises diabetic macroangiopathy, diabetic microangiopathy, impaired wound healing and diabetic ulcer. The terms "micro- and macrovascular diseases" and "micro- and macrovascular complications" are used interchangeably in this application.

According to another aspect of the disclosure, there is provided a method for preventing, slowing the progression of, delaying or treating a metabolic disorder selected from the group consisting of type 2 diabetes mellitus, impaired glucose tolerance (IGT), impaired fasting blood glucose (IFG), hyperglycemia, postprandial hyperglycemia, overweight, obesity, metabolic syndrome, gestational diabetes and diabetes related to cystic fibrosis in a patient in need thereof characterized in that the SGLT2 inhibitor and an insulin are administered, for example in combination or alternation, to the patient.

According to another aspect of the disclosure, there is provided a method for improving glycemic control and/or for reducing of fasting plasma glucose, of postprandial plasma glucose and/or of glycosylated hemoglobin HbA1c in a patient in need thereof characterized in that the SGLT2 inhibitor and an insulin are administered, for example in combination or alternation, to the patient.

The pharmaceutical composition according to this invention may also have valuable disease-modifying properties with respect to diseases or conditions related to impaired glucose tolerance (IGT), impaired fasting blood glucose (IFG), insulin resistance and/or metabolic syndrome.

According to another aspect of the disclosure, there is provided a method for preventing, slowing, delaying or reversing progression from impaired glucose tolerance (IGT), impaired fasting blood glucose (IFG), insulin resistance and/or from metabolic syndrome to type 2 diabetes mellitus in a patient in need thereof characterized in that the SGLT2 inhibitor and an insulin are administered, for example in combination or alternation, to the patient.

As by the use of a pharmaceutical composition according to this invention, an improvement of the glycemic

control in patients in need thereof is obtainable, also those conditions and/or diseases related to or caused by an increased blood glucose level may be treated.

By the administration of a pharmaceutical composition according to this invention and due to the activity of the SGLT2 inhibitor excessive blood glucose levels are not converted to insoluble storage forms, like fat, but excreted through the urine of the patient. In animal models using a SGLT2 inhibitor it can be seen that loss of fat accounts for the majority of the observed weight loss whereas no significant changes in body water or protein content are observed. Therefore, no or less gain in weight or even a reduction in body weight is the result.

According to another aspect of the disclosure, there is provided a method for reducing body weight and/or body fat or preventing or attenuating an increase in body weight and/or body fat or facilitating a reduction in body weight and/or body fat in a patient in need thereof characterized in that the SGLT2 inhibitor and an insulin are administered, for example in combination or alternation, to the patient.

By the administration of a combination or pharmaceutical composition according to the present invention, an abnormal accumulation of ectopic fat, in particular of the liver, may be reduced or inhibited. Therefore, according to another aspect of the present disclosure, there is provided a method for preventing, slowing, delaying or treating diseases or conditions attributed to an abnormal accumulation of ectopic fat, in particular of the liver, in a patient in need thereof characterized in that the SGLT2 inhibitor and an insulin are administered, for example in combination or alternation, to the patient. Diseases or conditions which are attributed to an abnormal accumulation of liver fat are particularly selected from the group consisting of general fatty liver, non-alcoholic fatty liver (NAFL), non-alcoholic steatohepatitis (NASH), hyperalimentation-induced fatty liver, diabetic fatty liver, alcoholic-induced fatty liver or toxic fatty liver.

As a result thereof, another aspect of the disclosureprovides a method for maintaining and/or improving the insulin sensitivity and/or for treating or preventing hyperinsulinemia and/or insulin resistance in a patient in need thereof characterized in that the SGLT2 inhibitor and an insulin are administered, for example in combination or alternation, to the patient.

According to another aspect of the disclosure, there is provided a method for preventing, slowing progression of, delaying, or treating new onset diabetes after transplantation (NODAT) and/or post-transplant metabolic syndrome (PTMS) in a patient in need thereof characterized in that the SGLT2 inhibitor and an insulin are administered, for example in combination or alternation, to the patient.

According to a further aspect of the disclosure, there is provided a method for preventing, delaying, or reducing NODAT and/or PTMS associated complications including micro- and macrovascular diseases and events, graft rejection, infection, and death in a patient in need thereof characterized in that the SGLT2 inhibitor and an insulin are administered, for example in combination or alternation, to the patient.

According to another aspect of the disclosure, there is provided a method for preventing, slowing progression of, delaying, or treating diabetes associated with cystic fibrosis in a patient in need thereof characterized in that the SGLT2 inhibitor and an insulin are administered, for example in combination or alternation, to the patient.

The pharmaceutical composition according to the invention is capable of facilitating the lowering of serum total urate levels in the patient. Therefore according to another aspect of the disclosure, there is provided a method for treating hyperuricemia and hyperuricemia-associated conditions, such as for example gout, hypertension and renal failure, in a patient in need thereof characterized in that the SGLT2 inhibitor and an insulin are administered, for example in combination or alternation, to the patient.

The administration of a pharmaceutical composition increases the urine excretion of glucose. This increase in osmotic excretion and water release and the lowering of urate levels are beneficial as a treatment or prevention for kidney stones. Therefore in a further aspect of the disclosure, there is provided a method for treating or preventing kidney stones in a patient in need thereof characterized in that the SGLT2 inhibitor and an insulin are administered, for example in combination or alternation, to the patient.

According to a further aspect of the disclosure, there is provided a method for treating hyponatremia, water retention and water intoxication in a patient in need thereof characterized in that the SGLT2 inhibitor and an insulin are administered, for example in combination or alternation, to the patient. By the administration of the pharmaceutical composition according to this invention it may be possible to reverse the effects of hyponatremia, water retention and water intoxication by acting on the kidney to reverse water retention and electrolyte imbalances associated with these diseases and disorders.

According to another aspect of the invention and disclosure there is provided the use of the SGLT2 inhibitor for the manufacture of a medicament for

- treating diabetes mellitus;
- treating diabetes mellitus, where treatment with insulin is required;
- treating type 1 diabetes mellitus;
- treating, preventing or reducing the risk of hypoglycemia;
- preventing, slowing progression of, delaying or treating of a condition or disorder selected from the group consisting of

complications of diabetes mellitus;

- preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity, metabolic syndrome and gestational diabetes; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance (IGT), impaired fasting blood glucose (IFG), insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, arteriosclerosis, myocardial infarction, stroke and peripheral arterial occlusive disease; or
- reducing body weight and/or body fat or preventing or attenuating an increase in body weight and/or body fat or facilitating a reduction in body weight and/or body fat; or
- preventing, slowing, delaying or treating diseases or conditions attributed to an abnormal accumulation of ectopic fat; or
- maintaining and/or improving the insulin sensitivity and/or for treating or preventing hyperinsulinemia and/or insulin resistance;
- preventing, slowing progression of, delaying, or treating new onset diabetes after transplantation (NODAT) and/or post-transplant metabolic syndrome (PTMS);
- preventing, delaying, or reducing NODAT and/or PTMS associated complications including micro- and macrovascular diseases and events, graft rejection, infection, and death;
- treating diabetes associated with cystic fibrosis
- treating hyperuricemia and hyperuricemia associated conditions;
- treating or prevention kidney stones;
- treating hyponatremia;

in a patient in need thereof characterized in that the SGLT2 inhibitor is administered, for example in combination or alternation, with an insulin.

According to another aspect of the invention and disclosure, there is provided the use of an insulin for the manufacture of a medicament for

- treating diabetes mellitus;
- treating diabetes mellitus, where treatment with insulin is required;
- treating type 1 diabetes mellitus;
- treating, preventing or reducing the risk of hypoglycemia;
- preventing, slowing progression of, delaying or treating of a condition or disorder selected from the group consisting of complications of diabetes mellitus;
- preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity and metabolic syndrome; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance (IGT), impaired fasting blood glucose (IFG), insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, arteriosclerosis, myocardial infarction, stroke and peripheral arterial occlusive disease; or
- reducing body weight and/or body fat or preventing or attenuating an increase in body weight and/or body fat or facilitating a reduction in body weight and/or body fat; or
- preventing, slowing, delaying or treating diseases or conditions attributed to an abnormal accumulation of liver fat; or
- maintaining and/or improving the insulin sensitivity and/or for treating or preventing hyperinsulinemia and/or insulin resistance;

in a patient in need thereof characterized in that the insulin is administered, for example in combination or alternation, with the SGLT2 inhibitor.

According to another aspect of the invention, there is provided the use of a pharmaceutical composition according to the present invention for the manufacture of a medicament for a therapeutic and preventive method as described hereinbefore and hereinafter.

## Definitions

The term **"active ingredient"** of a pharmaceutical composition according to the present invention means the SGLT2 inhibitor and/or the long acting insulin according to the present invention.

The term **"body mass index"** or **"BMI"** of a human patient is defined as the weight in kilograms divided by the square of the height in meters, such that BMI has units of $kg/m^2$.

The term **"overweight"** is defined as the condition wherein the individual has a BMI greater than or 25 $kg/m^2$ and less than 30 $kg/m^2$. The terms "overweight" and "pre-obese" are used interchangeably.

The term **"obesity"** is defined as the condition wherein the individual has a BMI equal to or greater than 30 $kg/m^2$. According to a WHO definition the term obesity may be categorized as follows: the term "class I obesity" is the condition wherein the BMI is equal to or greater than 30 $kg/m^2$ but lower than 35 $kg/m^2$; the term "class II obesity" is the condition wherein the BMI is equal to or greater than 35 $kg/m^2$ but lower than 40 $kg/m^2$; the term "class III obesity" is the condition wherein the BMI is equal to or greater than 40 $kg/m^2$.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women. With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term **"euglycemia"** is defined as the condition in which a subject has a fasting blood glucose concentration within the normal range, greater than 70 mg/dL (3.89 mmol/L) and less than 100 mg/dL (5.6 mmol/L). The word "fasting" has the usual meaning as a medical term.

The term **"hyperglycemia"** is defined as the condition in which a subject has a fasting blood glucose concentration above the normal range, greater than 100 mg/dL (5.6 mmol/L). The word "fasting" has the usual meaning as a medical term.

The term **"hypoglycemia"** is defined as the condition in which a subject has a blood glucose concentration below the normal range, in particular below 70 mg/dL (3.89 mmol/L) or even below 60 mg/dl.

The term **"postprandial hyperglycemia"** is defined as the condition in which a subject has a 2 hour postprandial blood glucose or serum glucose concentration greater than 200 mg/dL (11.1 mmol/L).

The term **"impaired fasting blood glucose"** or **"IFG"** is defined as the condition in which a subject has a fasting blood glucose concentration or fasting serum glucose concentration in a range from 100 to 125 mg/dl (i.e. from 5.6 to 6.9 mmol/l), in particular greater than 110 mg/dL and less than 126 mg/dl (7.00 mmol/L). A subject with "normal fasting glucose" has a fasting glucose concentration smaller than 100 mg/dl, i.e. smaller than 5.6 mmol/l.

The term **"impaired glucose tolerance"** or **"IGT"** is defined as the condition in which a subject has a 2 hour postprandial blood glucose or serum glucose concentration greater than 140 mg/dl (7.8 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.8 mmol/L).

The term **"hyperinsulinemia"** is defined as the condition in which a subject with insulin resistance, with or without euglycemia, has fasting or postprandial serum or plasma insulin concentration elevated above that of normal, lean individuals without insulin resistance, having a waist-to-hip ratio < 1.0 (for men) or < 0.8 (for women).

The terms "insulin-sensitizing", "insulin resistance-improving" or "insulin resistance-lowering" are synonymous and used interchangeably.

The term **"insulin resistance"** is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)"

score, a reliable indicator of insulin resistance (Katsuki A, et al. Diabetes Care 2001; 24: 362-5). Further reference is made to methods for the determination of the HOMA-index for insulin sensitivity *(*Matthews et al., Diabetologia 1985, 28: 412-19), of the ratio of intact proinsulin to insulin *(*Forst et al., Diabetes 2003, 52(Suppl.1): A459*)* and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):

$$\text{HOMA-IR} = [\text{fasting serum insulin } (\mu U/mL)] \times [\text{fasting plasma glucose}(mmol/L)/22.5]$$

As a rule, other parameters are used in everyday clinical practice to assess insulin resistance. Preferably, the patient's triglyceride concentration is used, for example, as increased triglyceride levels correlate significantly with the presence of insulin resistance.

Patients with a predisposition for the development of IGT or IFG or type 2 diabetes are those having euglycemia with hyperinsulinemia and are by definition, insulin resistant. A typical patient with insulin resistance is usually overweight or obese, but this is not always the case. If insulin resistance can be detected, this is a particularly strong indication of the presence of pre-diabetes. Thus, it may be that in order to maintain glucose homoeostasis a person have e.g. 2-3 times as high endogenous insulin production as a healthy person, without this resulting in any clinical symptoms.

The methods to investigate the **function of pancreatic beta-cells** are similar to the above methods with regard to insulin sensitivity, hyperinsulinemia or insulin resistance: An improvement of beta-cell function can be measured for example by determining a HOMA-index for beta-cell function *(*Matthews et al., Diabetologia 1985, 28: 412-19), the ratio of intact proinsulin to insulin *(*Forst et al., Diabetes 2003, 52(Suppl. 1): A459*),* the insulin/C-peptide secretion after an oral glucose tolerance test or a meal tolerance test, or by employing a hyperglycemic clamp study and/or minimal modeling after a frequently sampled intravenous glucose tolerance test *(*Stumvoll et al., Eur J Clin Invest 2001, 31: 380-81*).*

The term **"pre-diabetes"** is the condition wherein an individual is pre-disposed to the development of type 2 diabetes. Pre-diabetes extends the definition of impaired glucose tolerance to include individuals with a fasting blood glucose within the high normal range ≥ 100 mg/dL (J. B. Meigs, et al. Diabetes 2003; 52:1475-1484) and fasting hyperinsulinemia (elevated plasma insulin concentration). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

Individuals likely to have insulin resistance are those who have two or more of the following attributes: 1) overweight or obese, 2) high blood pressure, 3) hyperlipidemia, 4) one or more 1$^{st}$ degree relative with a diagnosis of IGT or IFG or type 2 diabetes. Insulin resistance can be confirmed in these individuals by calculating the HOMA-IR score. For the purpose of this invention, insulin resistance is defined as the clinical condition in which an individual has a HOMA-IR score > 4.0 or a HOMA-IR score above the upper limit of normal as defined for the laboratory performing the glucose and insulin assays.

The term **"type 1 diabetes"** is defined as the condition in which a subject has, in the presence of autoimmunity towards the pancreatic beta-cell or insulin, a fasting blood glucose or serum glucose concentration greater than 125 mg/dL (6.94 mmol/L). If a glucose tolerance test is carried out, the blood sugar level of a diabetic will be in excess of 200 mg of glucose per dL (11.1 mmol/l) of plasma 2 hours after 75 g of glucose have been taken on an empty stomach, in the presence of autoimmunity towards the pancreatic beta cell or insulin. In a glucose tolerance test 75 g of glucose are administered orally to the patient being tested after 10-12 hours of fasting and the blood sugar level is recorded immediately before taking the glucose and 1 and 2 hours after taking it. The presence of autoimmunity towards the pancreatic beta-cell may be observed by detection of circulating islet cell autoantibodies ["type 1A diabetes mellitus"], i.e., at least one of: GAD65 [glutamic acid decarboxylase-65], ICA [islet-cell cytoplasm], IA-2 [intracytoplasmatic domain of the tyrosine phosphatase-like protein IA-2], ZnT8 [zinc-transporter-8] or anti-insulin; or other signs of autoimmunity without the presence of typical circulating autoantibodies [type 1B diabetes], i.e. as detected through pancreatic biopsy or imaging). Typically a genetic predisposition is present (e.g. HLA, *INS* VNTR and *PTPN22),* but this is not always the case.

The term **"type 2 diabetes"** is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL (11.1 mmol/l) of plasma 2 hours after 75 g of glucose have been taken on an empty stomach. In a glucose tolerance test 75 g of glucose are administered orally to the patient being tested after 10-12 hours of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a secondary drug failure, indication for

insulin therapy and progression to micro- and macrovascular complications e.g. diabetic nephropathy, or coronary heart disease (CHD).

The term **"HbA1c"** refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients whose HbA1c value is consistently well adjusted by intensive diabetes treatment (i.e. < 6.5 % of the total haemoglobin in the sample), are significantly better protected against diabetic microangiopathy. For example, metformin on its own achieves an average improvement in the HbA1c value in the diabetic of the order of 1.0 - 1.5 %. This reduction of the HbA1C value is not sufficient in all diabetics to achieve the desired target range of < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The **"metabolic syndrome",** also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:

1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥ 150 mg/dL
3. HDL-cholesterol < 40 mg/dL in men
4. Blood pressure ≥ 130/85 mm Hg (SBP ≥ 130 or DBP ≥ 85)
5. Fasting blood glucose ≥ 100 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/-Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The definitions of **NODAT** (new onset diabetes after transplantation) and **PTMS** (post-transplant metabolic syndrome) follow closely that of the American Diabetes Association diagnostic criteria for type 2 diabetes, and that of the International Diabetes Federation (IDF) and the American Heart Association/National Heart, Lung, and Blood Institute, for the metabolic syndrome. NODAT and/or PTMS are associated with an increased risk of micro- and macrovascular disease and events, graft rejection, infection, and death. A number of predictors have been identified as potential risk factors related to NODAT and/or PTMS including a higher age at transplant, male gender, the pre-transplant body mass index, pre-transplant diabetes, and immunosuppression.

The term **"gestational diabetes"** (diabetes of pregnancy) denotes a form of the diabetes which develops during pregnancy and usually ceases again immediately after the birth. Gestational diabetes is diagnosed by a screening test which is carried out between the 24th and 28th weeks of pregnancy. It is usually a simple test in which the blood sugar level is measured one hour after the administration of 50 g of glucose solution. If this 1 h level is above 140 mg/dl, gestational diabetes is suspected. Final confirmation may be obtained by a standard glucose tolerance test, for example with 75 g of glucose.

The term **"hyperuricemia"** denotes a condition of high serum total urate levels. In human blood, uric acid concentrations between 3.6 mg/dL (ca. 214 μmol/L) and 8.3 mg/dL (ca. 494 μmol/L) are considered normal by the American Medical Association. High serum total urate levels, or hyperuricemia, are often associated with several maladies. For example, high serum total urate levels can lead to a type of arthritis in the joints kown as gout. Gout is a condition created by a build up of monosodium urate or uric acid crystals on the articular cartilage of joints, tendons and surrounding tissues due to elevated concentrations of total urate levels in the blood stream. The build up of urate or uric acid on these tissues provokes an inflammatory reaction of these tissues. Saturation levels of uric acid in urine may result in kidney stone formation when the uric acid or urate crystallizes in the kidney. Additionally, high serum total urate levels are often associated with the so-called metabolic syndrome, including cardiovascular disease and hypertension.

The term **"hyponatremia"** denotes a condition of a positive balance of water with or without a deficit of sodium, which is recognized when the plasma sodium falls below the level of 135 mml/L. Hyponatremia is a condition which can occur in isolation in individuals that over-consume water; however, more often hyponatremia is a complication of medication or other underlying medical condition that leas to a diminished excretion of water. Hyponatremia may lead to water intoxication, which occurs when the normal tonicity of extracellular fluid falls below the safe limit, due to retention of excess water. Water intoxication is a potentially fatal disturbance in brain function. Typical symptoms of water intoxication include nausea, vomiting, headache and malaise.

The term **"SGLT2 inhibitor"** in the scope of the present invention relates to a compound, in particular to a glucopyranosyl-derivative, i.e. compound having a glucopyranosyl-moiety, which shows an inhibitory effect on the sodium-glucose transporter 2 (SGLT2), in particular the human SGLT2. The inhibitory effect on hSGLT2 measured as IC50 is prerably below 1000 nM, even more preferably below 100 nM, most preferably below 50 nM. IC50 values of SGLT2 inhibitors are usually above 0.01 nM, or even equal to or above 0.1 nM. The inhibitory effect on hSGLT2 can be determined by methods known in the literature, in particular as described in the application WO 2005/092877 or WO 2007/093610 (pages 23/24). The term "SGLT2 inhibitor" also comprises any pharmaceutically acceptable salts thereof, hydrates and solvates thereof, including the respective crystalline forms.

The term **"insulin"** in the scope of the present invention relates to insulin and insulin analogs being used in the therapy of patients, in particular humans, which includes normal insulin, human insulin, insulin derivatives, zinc insulins and insulin analogues, including formulations thereof with modified release profiles. in particular as used in the therapy of humans. The term "insulin" in the scope of the present invention covers the following types of insulins:

- rapid-acting insulins,
- short-acting insulins,
- intermediate-acting insulins,
- long-acting insulins,

and mixtures thereof, for example mixtures of short- or rapid-acting insulins with long-acting insulins. The term "insulin" in the scope of the present invention covers insulins which are administered to the patient via injection, via infusion, including pumps, via inhalation, via oral, via transdermal or other routes of administration.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating",** "preventivally treating" and "preventing" are used interchangeably and comprise a treatment of patients at risk to develop a condition mentioned hereinbefore, thus reducing said risk.

## Brief Description of the Figures

The Figure 1 shows the blood glucose line in rats after administration of a SGLT2 inhibitor, insulin glargine and a combination thereof.

The Figure 2 shows the blood glucose line in rats after administration of a low-dose insulin glargine, a high dose insulin glargine and a combination of a SGLT2 inhibitor with a low dose of insulin glargine.

The Figure 3 shows the blood glucose line in rats after administration of insulin glargine and a co-administration of a SGLT2 inhibitor after 120 minutes.

The Figure 4 shows the effect on body fat portion after implantation of insulin-releasing sticks and after administration of a SGLT2 inhibitor alone and in addition to the implants.

## Detailed Description

Embodiments and aspects hereinbefore and hereinafter apply as embodiments and aspects according to the invention as long as they are covered by the appended claims. Embodiments and aspects hereinbefore and hereinafter not covered by the appended claims apply as embodiments and aspects according to this disclosure. Any references in the description to methods of treatment refer to the particular compound or pharmaceutical composition for use in a method of treatment of the patient.

The aspects according to the present invention, in particular the pharmaceutical compositions, methods and uses, refer to the SGLT2 inhibitor and insulins. In the methods and uses according to this invention a third antidiabetic

agent may optionally be administered, i.e. the SGLT2 inhibitor and the insulin are administered in combination without a third antidiabetic agent or with a third antidiabetic agent.

SGLT2 inhibitors selected from glucopyranosyl-substituted benzene derivatives of the formula (I)

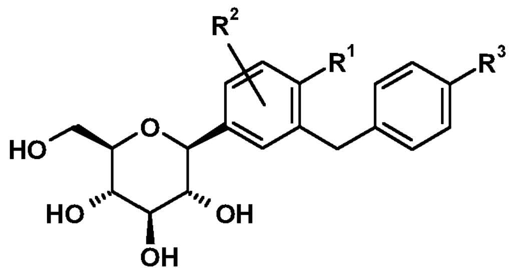

I

wherein $R^1$ denotes Cl, methyl or cyano; $R^2$ denotes H, methyl, methoxy or hydroxy and $R^3$ denotes ethyl, cyclopropyl, ethynyl, ethoxy, (R)-tetrahydrofuran-3-yloxy or (S)-tetrahydrofuran-3-yloxy and methods of their synthesis are described for example in the following patent applications: WO 2005/092877, WO 2006/117360, WO 2006/117359, WO 2006/120208, WO 2006/064033, WO 2007/031548, WO 2007/093610, WO 2008/020011, WO 2008/055870.

The SGLT2 inhibitor according to the present invention is the compound of the formula (I.9)

| | |
|---|---|
| (I.9) | 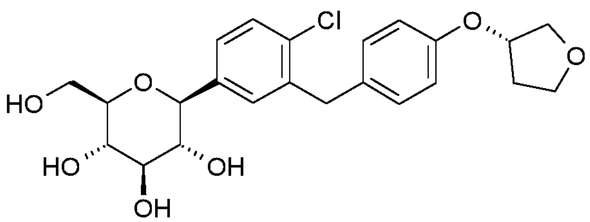 1-chloro-4-(β-D-glucopyranos-1-yl)-2-[4-(*(S)*-tetrahydrofuran-3-yloxy)-benzyl]-benzene, |

According to this invention and disclosure, it is to be understood that the definitions of the above listed SGLT2 inhibitors, including the glucopyranosyl-substituted benzene derivatives of the formula (I), also comprise their hydrates, solvates and polymorphic forms thereof, and prodrugs thereof. With regard to the compound (I.9) an advantageous crystalline form is described in the international patent application WO 2006/117359These crystalline forms possess good solubility properties which enable a good bioavailability of the SGLT2 inhibitor. Furthermore, the crystalline forms are physico-chemically stable and thus provide a good shelf-life stability of the pharmaceutical composition.

The aspects according to the present invention, in particular the pharmaceutical compositions, methods and uses, refer to an insulin, which includes normal insulin, human insulin, insulin derivatives, zinc insulins and insulin analogues, including formulations thereof with modified release profiles. in particular as used in the therapy of humans. The insulin may be selected from the group consisting of:

- rapid-acting insulins,
- short-acting insulins,
- intermediate-acting insulins,
- long-acting insulins,

and mixtures thereof.

Mixtures of insulins may comprise mixtures of short- or rapid-acting insulins with long-acting insulins. For example such mixtures are marketed as Actraphane/Mixtard or Novomix.

The term "insulin" in the scope of the present invention covers insulins as described hereinbefore and hereinafter which are administered to the patient via injection, preferably subcutaneous injection, via infusion, including pumps, via inhalation or other routes of administration. Insulins to be administered via inhalation are for example Exubera (Pfizer), AIR (Lilly) and AER (Novo Nordisk).

Rapid-acting insulins usually start lowering the blood glucose within about 5 to 15 minutes and are effective for about 3 to 4 hours. Examples of rapid-acting insulins are insulin aspart, insulin lispro and insulin glulisine. Insulin Lispro is marketed under the trade name Humalog and Liprolog. Insulin Aspart is marketed under the trade names NovoLog and NovoRapid. Insulin glulisine is marketed under the trade name Apidra.

Short-acting insulins usually start lowering the blood glucose within about 30 minutes and are effective about 5 to

8 hours. An example is regular insulin or human insulin.

Intermediate-acting insulins usually start lowering the blood glucose within about 1 to 3 hours and are effective for about 16 to 24 hours. An example is NPH insulin, also known as Humulin N, Novolin N, Novolin NPH and isophane insulin. Another example are lente insulins, such as Semilente or Monotard.

Long-acting insulins usually start lowering the blood glucose within 1 to 6 hours and are effective for up to about 24 hours or even up to or beyond 32 hours. Long-acting insulin usually provides a continuous level of insulin activity (for up to 24-36 hours) and usually operates at a maximum strength (with flat action profile) after about 8-12 hours, sometimes longer. Long-acting insulin is usually administered in the morning or before bed. Examples of long-acting insulin may include, but are not limited to, insulin glargine, insulin detemir or insulin degludec, which are insulin analogues, and ultralente insulin, which is regular human insulin formulated for slow absorption. Long-acting insulin is suited to provide for basal, as opposed to prandial, insulin requirements (e.g. to control hyperglycemia). Long-acting insulin may be typically administered ranging from twice or once daily, over thrice weekly up to once weekly (ultra long-acting insulin). Insulin glargine is marketed under the trade name Lantus for example. Insulin detemir is marketed under the tradename Levemir for example.

In one embodiment, the long-acting insulin of this invention refers to any insulin known in the art which is used for a basal insulin therapy and which have a basal release profile. A basal release profile refers to the kinetic, amount and rate of release of the insulin from the formulation into a patient's systemic circulation. In a graph of the patient's mean plasma insulin levels over time, a basal release profile typically has a minimal peak (often referred to as "a peakless profile" or "flat profile") and slowly and continuously releases insulin for a prolonged period of time.

In a further embodiment, the long-acting insulin is an acylated derivative of human insulin. Acylated insulin derivatives may be such wherein a lipophilic group is attached to the lysine residue in position B29. A commercial product is Levemir® comprising $Lys^{B29}(N^{\varepsilon}$-tetradecanoyl) des(B30) human insulin (insulin detemir). Another example is $N^{\varepsilon B29}$-($N^{\alpha}$-($\omega$-carboxypentadecanoyl)-L-$\gamma$-glutamyl) des(B30) human insulin (insulin degludec).

In a further embodiment, the long-acting insulin is such comprising positively charged amino acids such as Arg attached to the C-terminal end of the B-chain. A commercial product is Lantus® (insulin glargine) comprising $Gly^{A21}$, $Arg^{B31}$, $Arg^{B32}$ human insulin.

According to one embodiment of the invention the insulin is selected from the group consisting of long acting insulins.

According to another embodiment the insulin is selected from the **group G2** consisting of the long acting insulins (L1) to (L7) as described hereinafter:

(L1): Insulin glargine

Insulin glargine (marketed as LANTUS® by Sanofi-Aventis) is approved and marketed for subcutaneous administration once a day. Insulin glargine provides relatively constant glucose lowering activity over a 24-hour period and may be administered any time during the day provided it is administered at the same time every day.

(L2): Insulin detemir

Insulin detemir (marketed as LEVEMIR® by Novo Nordisk) is approved and marketed for subcutaneous administration either twice a day or once a day, preferably with the evening meal or at bedtime.

(L3): Insulin degludec

Insulin degludec (NN1250) is a neutral, soluble ultra-long acting insulin with a duration of action more than 24 hours. Degludec has a very flat, predictable and smooth action profile. It is intended for subcutaneous administration once daily or less (e.g. three times a week).

(L4): Insulin lispro PEGylated

Insulin lispro PEGylated with high molecular weight poly(ethylene glycol) derivatives especially as disclosed in WO 2009/152128, such as e.g. the PEGylated insulin lispro compound of the formula P-[(A)-(B)], or a pharmaceutically acceptable salt thereof, wherein A is the A-chain of insulin lispro, B is the B-chain of insulin lispro, and P is a PEG having a molecular weight in the range from about 17,5 kDa to about 40 kDa, and wherein A and B are properly cross-linked and P is attached via a urethane covalent bond to the epsilon-amino group of the lysine at position 28 of B.

(L5): Amidated insulin glargine

Amidated insulin glargine especially in the form of $Gly^{A21}$, $Arg^{B31}$, $Arg^{B32}$-$NH_2$ human insulin (insulin glargine amide, i.e. the C-terminus of the B-chain of insulin glargine is amidated) as disclosed in WO 2008/006496 or WO 2008/006496.

(L6):

$Lys^{B29}$($N^{\varepsilon}$-lithocholyl-$\gamma$-Glu) des(B30) human insulin or $N^{\varepsilon B29}$-$\omega$-carboxypentadecanoyl-$\gamma$-amino-butanoyl des(B30) human insulin.

(L7):
Amidated insulin analogs as disclosed in WO 2009/087082, especially one selected from claim 14, or in WO 2009/087081, especially one selected from claim 16.

Preferred members of the group G2 are L1, L2 and L3, in particular insulin glargine.

Long-acting insulins analogues are typically given as basic anti-diabetic therapy to type 1 diabetes, type 2 diabetes or latent autoimmune diabetes with onset in adults (LADA) patients to control the blood sugar when no food intake occurs. As mentioned above, this type of insulin provides a continuous level of insulin activity for up to 36 hours. Long-acting insulin operates at maximum strength after about 8-12 hours. Because of their advantages, it is thought that treatment with these insulin analogues can lead to a beneficial effect, for example less hypoglycaemia, less weight gain or a better metabolic control possibly resulting in less late diabetic complications such as problems with eyes, kidneys or feet and myocardial infarction, stroke or death.

According to the invention, there are provided methods for treating a patient with regard to diseases and conditions as described hereinbefore and hereinafter characterized in that the SGLT2 inhibitor and an insulin are administered, for example in combination or alternation, to the patient. Said diseases and conditions comprise diabetes mellitus, type 1 diabetes mellitus, type 2 diabetes mellitus, diseases which require treatment with insulin, conditions which require treatment with insulin, inter alia. According to one embodiment of the invention the insulin is part of a basal insulin therapy.

The term basal insulin therapy relates to a therapy in which one or more insulins are administered to a patient such that in a graph of the patient's mean plasma insulin levels over time, a basal release profile typically has a minimal peak (often referred to as "a peakless profile" or "flat profile") and slowly and continuously releases insulin for a prolonged period of time. According to one embodiment the basal insulin therapy includes the administration of a long-acting insulins to a patient. According to another embodiment the basal insulin therapy includes the administration of an insulin, in particular a rapid-acting or short-acting insulin, including human insulin, to a patient via infusion, for example via a pump in order to achieve the desired patient's mean plasma insulin level for a prolonged period of time, for example over 12 or 24 hours or longer.

Therefore according to one embodiment of the invention there is provided a method for treating a disease or condition selected from the group consisting of diabetes mellitus, type 1 diabetes mellitus, type 2 diabetes mellitus and a disease or condition which requires treatment with insulin in a patient characterized in that the patient receives a basal insulin therapy and in addition the SGLT2 inhibitor is administered to the patient.

According to one aspect of this embodiment the patient receives a basal insulin therapy wherein a long-acting insulin is administered to the patient. For example the long-acting insulin is administered via injection, for example subcutaneous injection. Preferably the SGLT2 inhibitor is administered orally. According to this aspect the long-acting insulin and the SGLT2 inhibitor are administered in combination or alternation, i.e. at the same time or at different times. For example the long-acting insulin is administered to the patient once or twice, preferably once daily. For example the SGLT2 inhibitor is administered to the patient once or twice, preferably once daily.

According to another aspect of this embodiment the patient receives a basal insulin therapy wherein an insulin is administered to the patient via infusion, for example via a pump. According to this aspect the insulin may be a rapid-acting or short-acting insulin, for example a human insulin. Preferably the SGLT2 inhibitor is administered orally. According to this aspect the insulin and the SGLT2 inhibitor are administered in combination or alternation, i.e. at the same time or at different times. For example the insulin is administered to the patient several times daily via pump infusion wherein the time and dose are chosen in order to achieve a certain range of plasma insulin level. For example the SGLT2 inhibitor is administered to the patient once or twice, preferably once daily.

According to another embodiment of the present invention the pharmaceutical composition, the methods and uses according to the invention additionally comprise a further antidiabetic agent.

A further antidiabetic agent is selected from the **group G3** consisting of biguanides, thiazolidindiones, sulfonylureas, glinides, inhibitors of alpha-glucosidase, GLP-1 analogues, DPP-4 inhibitors and amylin analogs, including pharmaceutically acceptable salts of the beforementioned agents. In the following preferred embodiments regarding the third antidiabetic agent are described.

The group G3 comprises biguanides. Examples of biguanides are metformin, phenformin and buformin. A preferred biguanide is metformin.

The term "metformin" as employed herein refers to metformin or a pharmaceutically acceptable salt thereof such as the hydrochloride salt, the metformin (2:1) fumarate salt, and the metformin (2:1) succinate salt, the hydrobromide salt, the p-chlorophenoxy acetate or the embonate, and other known metformin salts of mono and dibasic carboxylic acids. It is preferred that the metformin employed herein is the metformin hydrochloride salt.

The group G3 comprises thiazolidindiones. Examples of thiazolidindiones (TZD) are pioglitazone and rosiglitazone.

The term "pioglitazone" as employed herein refers to pioglitazone, including its enantiomers, mixtures thereof and its racemate, or a pharmaceutically acceptable salt thereof such as the hydrochloride salt.

The term "rosiglitazone" as employed herein refers to rosiglitazone, including its enantiomers, mixtures thereof and its racemate, or a pharmaceutically acceptable salt thereof such as the maleate salt.

The group G3 comprises sulfonylureas. Examples of sulfonylureas are glibenclamide, tolbutamide, glimepiride, glipizide, gliquidone, glibornuride, glyburide, glisoxepide and gliclazide. Preferred sulfonylureas are tolbutamide, gliquidone, glibenclamide, glipizide and glimepiride, in particular glibenclamide, glipizide and glimepiride.

Each term of the group "glibenclamide", "glimepiride", "gliquidone", "glibornuride", "gliclazide", "glisoxepide", "tolbutamide" and "glipizide" as employed herein refers to the respective active drug or a pharmaceutically acceptable salt thereof.

The group G3 comprises glinides. Examples of glinides are nateglinide, repaglinide and mitiglinide.

The term "nateglinide" as employed herein refers to nateglinide, including its enantiomers, mixtures thereof and its racemate, or a pharmaceutically acceptable salts and esters thereof.

The term "repaglinide" as employed herein refers to repaglinide, including its enantiomers, mixtures thereof and its racemate, or a pharmaceutically acceptable salts and esters thereof.

The group G3 comprises inhibitors of alpha-glucosidase. Examples of inhibitors of alpha-glucosidase are acarbose, voglibose and miglitol.

Each term of the group "acarbose", "voglibose" and "miglitol" as employed herein refers to the respective active drug or a pharmaceutically acceptable salt thereof.

The group G3 comprises inhibitors of GLP-1 analogues. Examples of GLP-1 analogues are exenatide and liraglutide.

Each term of the group "exenatide" and "liraglutide" as employed herein refers to the respective active drug or a pharmaceutically acceptable salt thereof.

The group G3 comprises inhibitors of DPP-4 inhibitors. Examples of DPP-4 inhibitors are linagliptin, sitagliptin, vildagliptin, saxagliptin, denagliptin, alogliptin, carmegliptin, melogliptin, dutogliptin, including pharmaceutically acceptable salts thereof, hydrates and solvates thereof.

The group G3 comprises amylin analogs. An example of an amylin analog is pramlintide, including pharmaceutically acceptable salts thereof, hydrates and solvates thereof. For example pramlintide acetate is marketed under the tradename Symlin.

According to an embodiment the pharmaceutical compositions, methods and uses according to the present invention relate to a combination of the SGLT2 inhibitor and an insulin which is preferably selected from the group of sub-embodiments E27 to E36 according to the entries in the Table 1.

Table 1

| Embodiment | SGLT2 Inhibitor | Insulin |
|---|---|---|
| E27 | Compound (I.9) | insulin |
| E28 | Compound (I.9) | rapid-acting or short-acting insulin |
| E29 | Compound (I.9) | human insulin |
| E30 | Compound (I.9) | intermediate-acting or long-acting insulin |
| E31 | Compound (I.9) | long-acting insulin |
| E32 | Compound (I.9) | selected from the group G2 |
| E33 | Compound (I.9) | L1 |
| E34 | Compound (I.9) | L2 |
| E35 | Compound (I.9) | L3 |
| E36 | Compound (I.9) | L4 |
| E37 | Compound (I.9) | L5 |
| E38 | Compound (I.9) | L6 |
| E39 | Compound (I.9) | L7 |

Among the combinations according to the present invention listed in Table 1, the combination according to the

entries E27 to E39 are even more preferred.

According to a further embodiment the pharmaceutical compositions, methods and uses according to the present invention relate to a combination of the SGLT2 inhibitor and an insulin which additionally comprises a further antidiabetic agent. Preferred sub-embodiments are selected from the entries in the Table 2.

Table 2

| Embodiment | SGLT2 Inhibitor | Insulin | Further antidiabetic agent |
|---|---|---|---|
| F12 | Compound (I.9) | insulin | metformin |
| F13 | Compound (I.9) | rapid-acting or short-acting insulin | metformin |
| F14 | Compound (I.9) | human insulin | metformin |
| F15 | Compound (I.9) | intermediate-acting or long-acting insulin | metformin |
| F16 | Compound (I.9) | long-acting insulin | metformin |
| F17 | Compound (I.9) | selected from the group G2 | metformin |
| F18 | Compound (I.9) | L1 | metformin |
| F19 | Compound (I.9) | L2 | metformin |
| F20 | Compound (I.9) | L3 | metformin |
| F21 | Compound (I.9) | L4 | metformin |
| F22 | Compound (I.9) | L5 | metformin |
| F23 | Compound (I.9) | L6 | metformin |
| F24 | Compound (I.9) | L7 | metformin |
| F36 | Compound (I.9) | insulin | pioglitazone |
| F37 | Compound (I.9) | rapid-acting or short-acting insulin | pioglitazone |
| F38 | Compound (I.9) | human insulin | pioglitazone |
| F39 | Compound (I.9) | intermediate-acting or long-acting insulin | pioglitazone |
| F40 | Compound (I.9) | long-acting insulin | pioglitazone |
| F41 | Compound (I.9) | selected from the group G2 | pioglitazone |
| F42 | Compound (I.9) | L1 | pioglitazone |
| F43 | Compound (I.9) | L2 | pioglitazone |
| F44 | Compound (I.9) | L3 | pioglitazone |
| F45 | Compound (I.9) | L4 | pioglitazone |
| F46 | Compound (I.9) | L5 | pioglitazone |
| F47 | Compound (I.9) | L6 | pioglitazone |
| F48 | Compound (I.9) | L7 | pioglitazone |
| F60 | Compound (I.9) | insulin | linagliptin |
| F61 | Compound (I.9) | rapid-acting or short-acting insulin | linagliptin |
| F62 | Compound (I.9) | human insulin | linagliptin |
| F63 | Compound (I.9) | intermediate-acting or long-acting insulin | linagliptin |
| F64 | Compound (I.9) | long-acting insulin | linagliptin |
| F65 | Compound (I.9) | selected from the group G2 | linagliptin |
| F66 | Compound (I.9) | L1 | linagliptin |
| F67 | Compound (I.9) | L2 | linagliptin |
| F68 | Compound (I.9) | L3 | linagliptin |
| F69 | Compound (I.9) | L4 | linagliptin |

(continued)

| Embodiment | SGLT2 Inhibitor | Insulin | Further antidiabetic agent |
|---|---|---|---|
| F70 | Compound (I.9) | L5 | linagliptin |
| F71 | Compound (I.9) | L6 | linagliptin |
| F72 | Compound (I.9) | L7 | linagliptin |

The combination of the SGLT2 inhibitor and an insulin according to this invention significantly improves the glycemic control, in particular in patients as described hereinafter, compared with a monotherapy using either the SGLT2 inhibitor or an insulin alone, for example with a monotherapy of a long-acting insulin, such as insulin glargine. Furthermore the combination of the SGLT2 inhibitor and an insulin according to this invention allows a reduction of the dose of the insulin compared with a monotherapy of said insulin, for example with a monotherapy of a long-acting insulin, such as insulin glargine. With a reduction of the dose of the insulin any side effects associated with the therapy using said insulin may be prevented or attenuated. A dose reduction is beneficial for patients which otherwise would potentially suffer from side effects in a therapy using a higher dose of one or more of the active ingredients, in particular with regard to side effect caused by the insulin. Therefore, the pharmaceutical composition as well as the methods according to the present invention, show less side effects, thereby making the therapy more tolerable and improving the patients compliance with the treatment. In addition the efficacy of the insulin, for example in a basal insulin therapy with a long acting insulin or with a short- or rapid acting insulin, including human insulin, via infusion with a pump, may be prolonged by a combined treatment with the SGLT-2 inhibitor. Therefore the time interval between two applications, for example subcutaneous injections or infusions via a pump, of the insulin may be prolonged. For example in a combination therapy employing a long acting insulin and the SGLT2 inhibitor according to the invention the dose of the long acting insulin, the dose of the SGLT2 inhibitor, the time intervall between two applications of the long acting insulin and the time intervall between the application of the long acting insuin and the SGLT2 inhibitor are chosen such that a good glycemic control is provided to the patient for a given time period, in particular for 24 hours.

When this invention refers to patients requiring treatment or prevention, it relates primarily to treatment and prevention in humans, but the pharmaceutical composition may also be used accordingly in veterinary medicine in mammals. In the scope of this invention the term "patient" covers adult humans (age of 18 years or older), adolescent humans (age 10 to 17 years) and children (age 6-9 years).

Furthermore, the method and/or use according to this invention is advantageously applicable in those patients who show one, two or more of the following conditions:

(a) type 1 diabetes mellitus;
(b) need for treatment with insulin;
(c) latent autoimmune diabetes with onset in adults (LADA).

Furthermore, the method and/or use according to this invention is advantageously applicable in those patients who are or shall be treated with a insulin, for example with insulin glargine or detemir insulin, in particular in patients diagnosed with type 1 diabetes mellitus, and show one, two or more of the following conditions, including the risk to develop such conditions:

(d) nocturnal and/or early morning hypoglycemia;
(e) hypoglycemic episodes;
(f) hyperglycemic episodes;
(g) cardiac or cerebral complications;
(h) retinopathy, in particular proliferative retinopathy;
(i) injection site reactions, for example skin or subcutaneous tissue disorders.

As described hereinbefore by the administration of the pharmaceutical composition according to this invention and in particular in view of the high SGLT2 inhibitory activity of the SGLT2 inhibitors therein, excessive blood glucose is excreted through the urine of the patient, so that less or no gain in weight or even a reduction in body weight may result. Therefore, a treatment or prophylaxis according to this invention is advantageously suitable in those patients in need of such treatment or prophylaxis who are diagnosed of one or more of the conditions selected from the group consisting of overweight and obesity, in particular class I obesity, class II obesity, class III obesity, visceral obesity and abdominal obesity. In addition a treatment or prophylaxis according to this invention is advantageously suitable in those patients in which a weight increase is contraindicated. Any weight increasing effect in the therapy, for example due to the administration of the third antidiabetic agent, may be attenuated or even avoided thereby.

Therefore, according to a preferred embodiment of the present invention, there is provided a method for improving glycemic control and/or for reducing of fasting plasma glucose, of postprandial plasma glucose and/or of glycosylated hemoglobin HbA1c in a patient in need thereof who is diagnosed with impaired glucose tolerance (IGT), impaired fasting blood glucose (IFG) with insulin resistance, with metabolic syndrome and/or with type 1 diabetes mellitus or type 2 diabetes mellitus characterized in that the SGLT2 inhibitor and an insulin as defined hereinbefore and hereinafter are administered, for example in combination or alternation, to the patient.

According to another preferred embodiment of the present invention, there is provided a method for improving gycemic control in patients, in particular in adult patients, with type 1 or type 2 diabetes mellitus as an adjunct to diet and exercise.

It can be found that by using a pharmaceutical composition according to this invention, an improvement of the glycemic control can be achieved even in those patients who have insufficient glycemic control in particular despite treatment with an insulin, for example despite maximal recommended or tolerated dose of monotherapy with the insulin.

Furthermore, the pharmaceutical composition, the methods and uses according to this invention are particularly suitable in the treatment of patients who are diagnosed having one or more of the following conditions

(a) obesity (including class I, II and/or III obesity), visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL.

It is assumed that patients diagnosed with impaired glucose tolerance (IGT), impaired fasting blood glucose (IFG), with insulin resistance and/or with metabolic syndrome suffer from an increased risk of developing a cardiovascular disease, such as for example myocardial infarction, coronary heart disease, heart insufficiency, thromboembolic events. A glycemic control according to this invention may result in a reduction of the cardiovascular risks.

Furthermore, the pharmaceutical composition, the methods and uses according to this invention are particularly suitable in the treatment of patients after organ transplantation, in particular those patients who are diagnosed having one or more of the following conditions

(a) a higher age, in particular above 50 years,
(b) male gender;
(c) overweight, obesity (including class I, II and/or III obesity), visceral obesity and/or abdominal obesity,
(d) pre-transplant diabetes,
(e) immunosuppression therapy.

Furthermore, the pharmaceutical composition, the methods and the uses according to this invention are particularly suitable in the treatment of patients who are diagnosed having one or more of the following conditions:

(a) hyponatremia, in particular chronical hyponatremia;
(b) water intoxication;
(c) water retention;
(d) plasma sodium concentration below 135 mmol/L.

Furthermore, the pharmaceutical composition, the methods and uses according to this invention are particularly suitable in the treatment of patients who are diagnosed having one or more of the following conditions:

(a) high serum uric acid levels, in particular greater than 6.0 mg/dL (357 μmol/L);
(b) a history of gouty arthritis, in particular recurrent gouty arthritis;
(c) kidney stones, in particular recurrent kidney stones;
(d) a high propensity for kidney stone formation.

A pharmaceutical composition according to this invention, in particular due to the SGLT2 inhibitor exhibits a good safety profile. Therefore, a treatment according to this invention is advantageous in those patients for which a reduction of the dose of the insulin is recommended.

A pharmaceutical composition according to this invention is particularly suitable in the long term treatment or prophylaxis of the diseases and/or conditions as described hereinbefore and hereinafter, in particular in the long term glycemic control in patients with type 1 diabetes mellitus or type 2 diabetes mellitus.

The term "long term" as used hereinbefore and hereinafter indicates a treatment of or administration in a patient

within a period of time longer than 12 weeks, preferably longer than 25 weeks, even more preferably longer than 1 year.

Therefore, a particularly preferred embodiment of the present invention provides a method for therapy, preferably oral therapy, for improvement, especially long term improvement, of glycemic control in patients with type 1 diabetes mellitus.

Therefore, a particularly preferred embodiment of the present invention provides a method for therapy, preferably oral therapy, for improvement, especially long term improvement, of glycemic control in patients with type 2 diabetes mellitus, especially in patients with late stage type 2 diabetes mellitus, in particular in patients additionally diagnosed of overweight, obesity (including class I, class II and/or class III obesity), visceral obesity and/or abdominal obesity.

Unless otherwise noted, the combination therapy according to the invention may refer to first line, second line or third line therapy, or initial or add-on combination therapy or replacement therapy.

According to one embodiment the SGLT2 inhibitor and the insulin and optionally the further antidiabetic agent are administered in combination, i.e. simultaneously, for example in one single formulation or in two separate formulations or dosage forms, or in alternation, for example successively in two or three separate formulations or dosage forms. Hence, the administration of one combination partner, i.e. the SGLT2 inhibitor or the insulin, may be prior to, concurrent to, or subsequent to the administration of the other combination partner. In one embodiment, for the combination therapy according to this invention the SGLT2 inhibitor and the insulin are administered in different formulations or different dosage forms. In another embodiment, for the combination therapy according to this invention the SGLT2 inhibitor and the insulin are administered in the same formulation or in the same dosage form.

Therefore according to an embodiment of the present invention there is provided a pharmaceutical composition or fixed dose combination comprising

a) the SGLT2 inhibitor as defined herein, and
b) an insulin as defined herein,

and, optionally, one or more pharmaceutically acceptable carriers and/or diluents.

Within the scope of the present invention, the SGLT2 inhibitor is preferably administered orally or by injection, preferably orally. The insulin is preferably administered by injection, preferably subcutaneously, or by infusion, for example with a pump. Other forms of administration are possible and described hereinafter. Preferably the optionally administered other antidiabetic agent is administered orally. In this case the SGLT2 inhibitor and the other antidiabetic agent may be comprised together in one dosage form or in separate dosage forms.

Therefore according to another embodiment the present invention provides a pharmaceutical composition, delivery system or device for systemic use, in particular for administration by injection or infusion, for example subcutaneous injection or infusion via pump, comprising

a) the SGLT2 inhibitor as defined herein, and, optionally,
b) an insulin as defined herein,

and, optionally, one or more pharmaceutically acceptable carriers and/or diluents.

It will be appreciated that the amount of the SGLT2 inhibitor and the insulin and optionally of the further antidiabetic agent according to this invention to be administered to the patient and required for use in treatment or prophylaxis according to the present invention will vary with the route of administration, the nature and severity of the condition for which treatment or prophylaxis is required, the age, weight and condition of the patient, concomitant medication and will be ultimately at the discretion of the attendant physician. In general, however, the SGLT2 inhibitor, the insulin and optionally the further antidiabetic agent according to this invention are included in the pharmaceutical composition or dosage form in an amount sufficient that by their administration in combination and/or alternation the glycemic control in the patient to be treated is improved.

For the treatment of hyperuricemia or hyperuricemia associated conditions the SGLT2 inhibitor according to this invention is included in the pharmaceutical composition or dosage form in an amount sufficient that is sufficient to treat hyperuricemia without disturbing the patient's plasma glucose homeostasis, in particular without inducing hypoglycemia.

For the treatment or prevention of kidney stones the SGLT2 inhibitor according to this invention is included in the pharmaceutical composition or dosage form in an amount sufficient that is sufficient to treat or prevent kidney stones without disturbing the patient's plasma glucose homeostasis, in particular without inducing hypoglycemia.

For the treatment of hyponatremia and associated conditions the SGLT2 inhibitor according to this invention is included in the pharmaceutical composition or dosage form in an amount sufficient that is sufficient to treat hyponatremia or the associated conditions without disturbing the patient's plasma glucose homeostasis, in particular without inducing hypoglycemia.

In the following preferred ranges of the amount of the SGLT2 inhibitor, the insulin and optionally the further antidiabetic agent to be employed in the pharmaceutical composition and the methods and uses according to this invention

are described. These ranges refer to the amounts to be administered per day with respect to an adult patient, in particular to a human being, for example of approximately 70 kg body weight, and can be adapted accordingly with regard to an administration 1 or 2 times daily and with regard to other routes of administration and with regard to the age of the patient. The ranges of the dosage and amounts are calculated for the inidividual active moiety. Advantageously, the combination therapy according to the present invention utilizes lower dosages of the individual SGLT2 inhibitor, of the individual insulin and/or optionally of the individual further antidiabetic agent used in monotherapy or used in conventional therapeutics, thus avoiding possible adverse side effects incurred when those agents are used as monotherapies.

In general, the amount of the SGLT2 inhibitor in the pharmaceutical composition, methods and uses according to this invention is preferably in the range from 1/5 to 1/1 of the amount usually recommended for a monotherapy using said SGLT2 inhibitor.

The preferred dosage range of the SGLT2 inhibitor is in the range from 0.5 mg to 200 mg, even more preferably from 1 to 100 mg, most preferably from 1 to 50 mg per day. The oral administration is preferred. Therefore, a pharmaceutical composition may comprise the hereinbefore mentioned amounts, in particular from 1 to 50 mg or 1 to 25 mg. Particular dosage strengths (e.g. per tablet or capsule) are for example 1, 2.5, 5, 7.5, 10, 12.5, 15, 20, 25 or 50 mg of the compound of the formula (I), in particular of the compound (I.9), or of dapagliflozin. The application of the active ingredient may occur one, two or three times a day, preferably once a day.

In general, the amount of the insulin in the pharmaceutical composition, methods and uses according to this invention is preferably in the range from 1/5 to 1/1 of the amount usually recommended for a monotherapy using said long acting insulin.

The insulin is typically administered by subcutaneous injection, e.g. ranging from twice daily, once daily to once weekly injection. Suitable doses and dosage forms of the insulin may be determined by a person skilled in the art. Blood glucose monitoring is essential in all patients receiving insulin therapy. Doses of a long-acting insulin will be individualized accoring to the response to treatment and obtainment of glycaemic control. Doses are; typically in the range of 10 to 70 units/day. According to the WHO the defined daily dose of insulin is 40 units. Usually long acting insulins are given once daily, either in the morning or in the evening. An SGLT-2 inhibitor could be administerd at any of these time points. Type 1 diabetes patients would usually be treated with a multiple daily injection regimen comprising basal insulin, for example a long-acting insulin, and a rapid acting insulin. A typical daily insulin requirement in type 1 diabetes is 40 to 60 units, depending on beta-cell function, age, weight, degree of physical activity, eating and drinking. Typically around 40-60% of the total daily insulin requirement would be given as basal insulin, for example with a long-acting insulin.

For example, insulin glargine (Lantus) is administered subcutaneously once a day. Lantus may be administered at any time during the day, but at the same time every day. The dose of Lantus is individualized based on clinical response. A typical starting dose of Lantus in patients with type 2 diabetes who are not currently treated with insulin is 10 units, or alternatively 0.2 U/kg, once daily, which should subsequently be adjusted to the patient's needs. Type 1 diabetes patients would usually be treated with a multiple daily injection regimen comprising basal insulin and rapid acting insulin. A typical daily insulin requirement in type 1 diabetes is 40 to 60 units, depending on beta-cell function, age, weight, degree of physical activity, eating and drinking. Typically around 40-60% of the total daily insulin requirement would be given as basal insulin; when Lantus is used, the same principle for dosing applies in type 1 diabetes as in type 2 diabetes. Also here, a titration of insulin dosages are needed based on clinical response.

Insulin detemir (Levemir) is administered subcutaneously once or twice a day. For patients treated with Levemir once daily, the dose is preferably administered with the evening meal or at bedtime. For patients who require twice-daily dosing, the evening dose can be administered either with evening meal, at bedtime, or 12 hours after the morning dose. The dose of Levemir is individualized based on clinical response. For insulin-naive patients with type 2 diabetes who are inadequately controlled on oral antidiabetic drugs, Levemir should be started at a dose of 0.1 to 0.2 Units/kg once-daily in the evening or 10 units once- or twice-daily, and the dose adjusted to achieve glycemic targets. In type 1 diabetes patients would usually be treated with a multiple daily injection regimen comprising basal insulin and rapid acting insulin. A typical daily insulin requirement in type 1 diabetes is 40-60 units, depending on beta-cell function, age, weight, degree of physical activity, eating and drinking. Typically around 40-60% of the total daily insulin requirement would be given as basal insulin; when Levemir is used, the same principle for dosing applies in type 1 diabetes as in type 2 diabetes. Also here, a titration of insulin dosages are needed based on clinical response.

Furthermore the long acting insulin analogues such as insulin degludec and basal insulin lispro will be developed with a final formulation of U-100 and dosing will be individually adapted for these insulins as well, both in type 1 and type 2 diabetes..

In case the SGLT-2 inhibitor and the insulin are to be combined with a further antidiabetic agent, the dose of the further antidiabetic agent is preferably in the range from 1/5 to 1/1 of the dose usually recommended for a monotherapy using said further antidiabetic agent. Using lower dosages of the individual further antidiabetic agent compared with monotherapy could avoid or minimize possible toxicity and adverse side effects incurred when those agents are used as monotherapies.

With regard to metformin as a preferred further antidiabetic agent metformin is usually given in doses varying

from about 500 mg to 2000 mg up to 3000 mg per day using various dosing regimens from about 100 mg to 500 mg or 200 mg to 850 mg (1-3 times a day), or about 300 mg to 1000 mg once, twice or thrice a day, or delayed-release metformin in doses of about 100 mg to 1000 mg or preferably 500 mg to 1000 mg once or twice a day or about 500 mg to 2000 mg once a day. Particular dosage strengths may be 250, 500, 625, 750, 850 and 1000 mg of metformin hydrochloride.

For children 10 to 16 years of age, the recommended starting dose of metformin is 500 mg given once daily. If this dose fails to produce adequate results, the dose may be increased to 500 mg twice daily. Further increases may be made in increments of 500 mg weekly to a maximum daily dose of 2000 mg, given in divided doses (e.g. 2 or 3 divided doses). Metformin may be administered with food to decrease nausea.

With regard to pioglitazone as a preferred further antidiabetic agent a dosage of pioglitazone is usually of about 1-10 mg, 15 mg, 30 mg, or 45 mg once a day.

With regard to linagliptine as a preferred further antidiabetic agent a dosage of linagliptine is usually of about 1-10 mg, for example 1, 2.5, 5 or 10 mg once a day.

In the methods and uses according to the present invention the SGLT2 inhibitor and the insulin are administered in combination or alternation. The term "administration in combination" means that the active ingredients are administered at the same time, i.e. simultaneously, or essentially at the same time. The term "administration in alternation" means that at first one of the two active ingredients, i.e. the SGLT2 inhibitor or the insulin, is administered and after a period of time the other active ingredient, i.e. the insulin or the SGLT2 inhibitor, is administered, i.e. both active ingredients are administered sequentially. The period of time between the administration of the first and of the second active ingredient may be in the range from 1 min to 12 hours. The administration which is in combination or in alternation may be once, twice, three times or four times daily, preferably once or twice daily.

A pharmaceutical composition which is present as a separate or multiple dosage form, preferably as a kit of parts, is useful in combination therapy to flexibly suit the individual therapeutic needs of the patient.

According to a first embodiment a preferred kit of parts comprises

(a) a first containment containing a dosage form comprising the SGLT2 inhibitor and at least one pharmaceutically acceptable carrier, and
(b) a second containment containing a dosage form comprising the insulin and at least one pharmaceutically acceptable carrier.

According to a second embodiment a preferred kit of parts comprises

(a) a first containment containing a dosage form comprising the SGLT2 inhibitor and at least one pharmaceutically acceptable carrier, and
(b) a second containment containing a dosage form comprising the insulin and at least one pharmaceutically acceptable carrier, and
(b) a third containment containing a dosage form comprising a further antidiabetic agent (for example metformin, pioglitazone or linagliptine) and at least one pharmaceutically acceptable carrier.

According to a third embodiment a preferred kit of parts comprises

(a) a first containment containing a dosage form comprising the SGLT2 inhibitor and a further antidiabetic agent and at least one pharmaceutically acceptable carrier, and
(b) a second containment containing a dosage form comprising the insulin and at least one pharmaceutically acceptable carrier.

A further aspect of the present invention is a manufacture comprising the pharmaceutical composition being present as separate dosage forms according to the present invention and a label or package insert comprising instructions that the separate dosage forms are to be administered in combination or alternation.

According to a first embodiment a manufacture comprises (a) a pharmaceutical composition comprising the SGLT2 inhibitor according to the present invention and (b) a label or package insert which comprises instructions that the medicament may or is to be administered, for example in combination or alternation, with a medicament comprising an insulin according to the present invention or with a medicament comprising both a insulin and a further antidiabetic agent according to the present invention.

According to a second embodiment a manufacture comprises (a) a pharmaceutical composition comprising an insulin according to the present invention and (b) a label or package insert which comprises instructions that the medicament may or is to be administered, for example in combination or alternation, with a medicament comprising the SGLT2 inhibitor according to the present invention or with a medicament comprising both the SGLT2 inhibitor and a further antidiabetic agent according to the present invention.

According to a third embodiment a manufacture comprises (a) a pharmaceutical composition comprising the SGLT2 inhibitor and a further antidiabetic agent according to the present invention and (b) a label or package insert which comprises instructions that the medicament may or is to be administered, for example in combination or alternation, with a medicament comprising an insulin according to the present invention.

The desired dose of the pharmaceutical composition according to this invention may conveniently be presented in a once daily or as divided dose administered at appropriate intervals, for example as two, three or more doses per day.

The pharmaceutical composition may be formulated for oral, parenteral (including subcutaneous) or other routes of administration in liquid or solid form. Oral administration of the SGLT2 inhibitor is preferred. The formulations may, where appropriate, be conveniently presented in discrete dosage units and may be prepared by any of the methods well known in the art of pharmacy. All methods include the step of bringing into association the active ingredient with one or more pharmaceutically acceptable carriers, like liquid carriers or finely divided solid carriers or both, and then, if necessary, shaping the product into the desired formulation. Examples of pharmaceutical compositions comprising the SGLT2 inhibitor compound (I.9) are described in WO 2010/092126. Examples of pharmaceutical compositions comprising the SGLT2 inhibitor compound (I.9) and linagliptin are described in WO 2010/092124.

The pharmaceutical composition may be formulated in the form of solutions, suspensions, emulsions, tablets, granules, fine granules, powders, capsules, caplets, soft capsules, pills, oral solutions, syrups, dry syrups, chewable tablets, troches, effervescent tablets, drops, fast dissolving tablets, oral fast-dispersing tablets, etc.. Preferably the pharmaceutical composition of the SGLT2 inhibitor is in the form of tablets.

The pharmaceutical composition and the dosage forms preferably comprises one or more pharmaceutical acceptable carriers. Preferred carriers must be "acceptable" in the sense of being compatible with the other ingredients of the formulation and not deleterious to the recipient thereof. Examples of pharmaceutically acceptable carriers are known to the one skilled in the art.

The pharmaceutical composition according to the invention may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilisation from solution, for constitution with a suitable vehicle, e.g. sterile, pyrogen-free water, before use.

Injectable formulations of the insulin and/or the SGLT2 inhibitor of this invention (particularly for subcutaneous use) may be prepared according to known formulation techniques, e.g. using suitable liquid carriers, which usually comprise sterile water, and, optionally, further additives such as e.g. preservatives, pH adjusting agents, buffering agents, isotoning agents, solubility aids and/or tensides or the like, to obtain injectable solutions or suspensions. In addition, injectable formulations may comprise further additives, for example salts, solubility modifying agents or precipitating agents which retard release of the drug(s). In further addition, injectable insulin formulations may comprise insulin stabilizing agents, such as zinc compounds. The component insulin of the combination according to the invention is preferably administered by injection (preferably subcutaneously) or by infusion (for example using a pump or comparable delivery system).

For further details on dosage forms, formulations and administration of SGLT2 inhibitors of this invention and/or insulin of this invention, reference is made to scientific literature and/or published patent documents, particularly to those cited herein.

The pharmaceutical compositions (or formulations) may be packaged in a variety of ways. Generally, an article for distribution includes one or more containers that contain the one or more pharmaceutical compositions in an appropriate form. Tablets are typically packed in an appropriate primary package for easy handling, distribution and storage and for assurance of proper stability of the composition at prolonged contact with the environment during storage. Primary containers for tablets may be bottles or blister packs.

Solutions for injection may be available in typical suitable presentation forms such as vials, cartridges or prefilled (disposable) pens, which may be further packaged.

The article may further comprise a label or package insert, which refers to instructions customarily included in commercial packages of therapeutic products, that may contain information about the indications, usage, dosage, administration, contraindications and/or warnings concerning the use of such therapeutic products. In one embodiment, the label or package inserts indicates that the composition can be used for any of the purposes described hereinbefore or hereinafter.

The pharmaceutical compositions and methods according to this invention show advantageous effects in the treatment and prevention of those diseases and conditions as described hereinbefore compared with pharmaceutical compositions and methods which comprise only one of the two active ingredients. Additional advantageous effects may be seen for example with respect to efficacy, dosage strength, dosage frequency, pharmacodynamic properties, pharmacokinetic properties, fewer adverse effects, convenience, compliance, etc..

Methods for the manufacture of SGLT2 inhibitors according to this disclosure and invention and of prodrugs thereof are known to the one skilled in the art. Advantageously, the compounds according to this invention and disclosure can be prepared using synthetic methods as described in the literature, including patent applications as cited hereinbefore. Preferred methods of manufacture are described in the WO 2006/120208 and WO 2007/031548. With regard to the preferred compound (I.9) an advantageous crystalline form is described in the international patent application WO 2006/117359.

With respect to insulins the methods of synthesis are known to the skilled person and as described in the scientific literature and/ or in published patent documents, particularly in those cited hereinbefore.

The active ingredients, in particular the insulin and/or the further antidiabetic agent, may be present in the form of a pharmaceutically acceptable salt. The active ingredients or a pharmaceutically acceptable salt thereof may be present in the form of a solvate such as a hydrate or alcohol adduct.

Any of the above mentioned combinations and methods within the scope of the invention may be tested by animal models known in the art. In the following, *in vivo* experiments are described which are suitable to evaluate pharmacologically relevant properties of pharmaceutical compositions and methods according to this invention:

Pharmaceutical compositions and methods according to this invention can be tested in genetically hyperinsulinemic or diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

The effect on glycemic control of the combinations according to this invention can be tested after single dosing of the SGLT2 inhibitor and the insulin alone and in combination in an oral glucose tolerance test in the animal models described hereinbefore. The time course of blood glucose is followed after an oral glucose challenge in overnight fasted animals. The combinations according to the present invention significantly improve glucose excursion compared to each monotherapy as measured by reduction of peak glucose concentrations or reduction of glucose AUC. In addition, after multiple dosing of the SGLT2 inhibitor and the insulin alone and in combination in the animal models described hereinbefore, the effect on glycemic control can be determined by measuring the HbA1c value in blood. The combinations according to this invention significantly reduce HbA1c compared to each monotherapy.

The possible dose reduction of one or both of the SGLT2 inhibitor and the insulin can be tested by the effect on glycemic control of lower doses of the combinations and monotherapies in the animal models described hereinbefore. The combinations according to this invention at the lower doses significantly improve glycemic control compared to placebo treatment whereas the monotherapies at lower doses do not.

A superior effect of the combination of the SGLT2 inhibitor and a insulin according to the present invention on beta-cell regeneration and neogenesis can be determined after multiple dosing in the animal models described hereinbefore by measuring the increase in pancreatic insulin content, or by measuring increased beta-cell mass by morphometric analysis after immunohistochemical staining of pancreatic sections, or by measuring increased glucose-stimulated insulin secretion in isolated pancreatic islets.

## Pharmacological Examples

The following examples show the beneficial effect on glycemic control of the combination according to the present invention.

### Reference **Example 1a:**

The following example shows the beneficial effect on glycemic control of the combination of a SGLT2 inhibitor (compound (I.9)) and an insulin (insulin glargine) as compared to the respective monotherapies. All experimental protocols concerning the use of laboratory animals were reviewed by a federal Ethics Committee and approved by governmental authorities. Two weeks before the study starts, the rats were pretreated with a single dose of 60 mg/kg i.p. of streptozotocin to induced experimental diabetes, resembling a type 1 diabetic condition. During the study blood glucose was followed over 4 h in male, 3-h fasted Sprague-Dawley rats (Crl:CD) with an age of 8-9 weeks at the start of the study. A pre-dose blood sample was obtained by tail bleed for randomization and blood glucose was measured with a glucometer 30, 60, 90 min and 2, 3, 4 hours after administration of the insulin and/or the SGLT2 inhibitor. At time point 0 min, the animals (n = 4-6 per group) were injected either with insulin glargine or isotonic NaCl subcutaneously. Simultaneously all animals received oral administrations of either vehicle alone (0.5% aqueous hydroxyethylcellulose) or this vehicle containing the SGLT2 inhibitor. The data are presented as mean ± S.E.M. Statistical comparison was conducted by repeated measures two-way ANOVA (analysis of variance) followed by Bonferroni post tests for group-wise comparisons. A p value $< 0.05$ was considered to show a statistically significant difference. The result is shown in Figure 1a. The term "Cpd. A" denotes the SGLT2 inhibitor compound (I.9) at a dose of 10 mg/kg. Insulin glargine was administered at a dose of 1.5 IU/animal. The term "Cpd. A + Insulin glargine" denotes the combination of the SGLT2 inhibitor compound (I.9) and insulin glargine at the

same doses. P values versus control are indicated by asterisks and p values of the monotherapies versus the combination are indicated by crosses (one symbol, $p < 0.05$; two symbols, $p < 0.01$; three symbols, $p < 0.001$). Four hours after administration, the SGLT2 inhibitor had reduced blood glucose by 19% versus control, and insulin glargine by 27%. Both treatments did not show statistically significant differences versus control. The combination significantly decreased blood glucose by 53% versus control. The decreased blood glucose in the combination group was significantly different from the SGLT2 inhibitor monotherapy.

Reference **Example 1b:**

The following example shows the beneficial effect on glycemic control of the combination of a SGLT2 inhibitor (compound (I.9)) and an insulin (insulin glargine) as compared to the respective monotherapies. All experimental protocols concerning the use of laboratory animals were reviewed by a federal Ethics Committee and approved by governmental authorities. Two weeks before the study starts, the rats were pretreated with a single dose of 60 mg/kg i.p. of streptozotocin to induced experimental diabetes, resembling a type 1 diabetic condition. During the study blood glucose was followed over 6 h in male, 3-h fasted Sprague-Dawley rats (Crl:CD) with an age of 8-9 weeks at the start of the study. A pre-dose blood sample was obtained by tail bleed for randomization and blood glucose was measured with a glucometer 30, 60, 90 min and 2, 3, 4, 5, 6 hours after administration of insulin and/or the SGLT2 inhibitor. At time point 0 min, the animals (n = 4-6 per group) were injected either with insulin glargine or isotonic NaCl subcutaneously. Simultaneously all animals received oral administrations of either vehicle alone (0.5% aqueous hydroxyethylcellulose) or this vehicle containing the SGLT2 inhibitor. The data are presented as mean±S.E.M. Statistical comparison was conducted by repeated measures two-way ANOVA followed by Bonferroni post tests for group-wise comparisons. A p value $< 0.05$ was considered to show a statistically significant difference. The result is shown in Figure 1b. The term "Cpd. A" denotes the SGLT2 inhibitor compound (I.9) at a dose of 10 mg/kg. Insulin glargine was administered at a dose of 1.5 IU/animal. The term "Cpd. A + Insulin glargine" denotes the combination of the SGLT2 inhibitor compound (I.9) and insulin glargine at the same doses. P values versus control are indicated by asterisks and p values of the monotherapies versus the combination are indicated by crosses (one symbol, $p < 0.05$; two symbols, $p < 0.01$; three symbols, $p < 0.001$). Six hours after administration, the SGLT2 inhibitor had reduced blood glucose by 13% versus control, and insulin glargine by 22%. Both treatments did not show statistically significant differences versus control. The combination significantly decreased blood glucose by 49% versus control. The decreased blood glucose in the combination group was significantly different from the SGLT2 monotherapy.

Reference **Example 2a:**

The following example shows the beneficial effect on glycemic control of the combination of a SGLT2 inhibitor (compound (I.9)) and a low dose of an insulin (insulin glargine) as compared to a high dose of an insulin (insulin glargine). All experimental protocols concerning the use of laboratory animals were reviewed by a federal Ethics Committee and approved by governmental authorities. Two weeks before the study starts, the rats were pretreated with a single dose of 60 mg/kg i.p. of streptozotocin to induced experimental diabetes, resembling a type 1 diabetic condition. During the study blood glucose was followed over 4 h in male, 3-h fasted Sprague-Dawley rats (Crl:CD) with an age of 8-9 weeks at the start of the study. A pre-dose blood sample was obtained by tail bleed for randomization and blood glucose was measured with a glucometer 30, 60, 90 min and 2, 3, 4 h after administration of the insulin alone or together with the SGLT2 inhibitor. At time point 0 min, the animals (n = 4-6 per group) were injected either with insulin glargine or isotonic NaCl subcutaneously. Simultaneously all animals received oral administrations of either vehicle alone (0.5% aqueous hydroxyethylcellulose) or this vehicle containing the SGLT2 inhibitor. The data are presented as mean±S.E.M. Statistical comparison was conducted by repeated measures two-way ANOVA followed by Bonferroni post tests for group-wise comparisons. A p value $< 0.05$ was considered to show a statistically significant difference. The result is shown in Figure 2a. Insulin glargine was administered at a dose of 1.5 IU/animal (low-dose) or 6 IU/animal (high-dose). The term "Cpd. A + low-dose insulin glargine" denotes the combination of the SGLT2 inhibitor at a dose of 10 mg/kg and insulin glargine at a dose of 1.5 IU/animal. P values versus control are indicated by asterisks and p values of the low-dose insulin glargine versus the combination or the high-dose insuline glargine are indicated by crosses (one symbol, $p < 0.05$; two symbols, $p < 0.01$; three symbols, $p < 0.001$). Four hours after administration, the low-dose insuline glargine had reduced blood glucose by 27% versus control without showing statistically significant difference. The combination had decreased blood glucose by 53%, which was in the range of the high-dose insulin glargine with a decrease of 47%. Both treatments were significantly different from the control.

Reference **Example 2b:**

The following example shows the beneficial effect on glycemic control of the combination of a SGLT2 inhibitor

(compound (I.9)) and a low dose of an insulin (insulin glargine) as compared to a high dose of an insulin (insulin glargine). All experimental protocols concerning the use of laboratory animals were reviewed by a federal Ethics Committee and approved by governmental authorities. Two weeks before the study starts, the rats were pretreated with a single dose of 60 mg/kg i.p. of streptozotocin to induced experimental diabetes, resembling a type 1 diabetic condition. During the study blood glucose was followed over 6 h in male, 3-h fasted Sprague-Dawley rats (Crl:CD) with an age of 8-9 weeks at the start of the study. A pre-dose blood sample was obtained by tail bleed for randomization and blood glucose was measured with a glucometer 30, 60, 90 min and 2, 3, 4, 5, 6 h after administration of insulin alone or together with the SGLT2 inhibitor. At time point 0 min, the animals (n = 4-6 per group) were injected either with insulin glargine or isotonic NaCl subcutaneously. Simultaneously all animals received oral administrations of either vehicle alone (0.5% aqueous hydroxyethylcellulose) or this vehicle containing the SGLT2 inhibitor. The data are presented as mean±S.E.M. Statistical comparison was conducted by repeated measures two-way ANOVA followed by Bonferroni post tests for group-wise comparisons. A p value < 0.05 was considered to show a statistically significant difference. The result is shown in Figure 2b. Insulin glargine was administered at a dose of 1.5 IU/animal (low-dose) or 6 IU/animal (high-dose). The term "Cpd. A + low-dose insulin glargine" denotes the combination of the SGLT2 inhibitor at a dose of 10 mg/kg and insulin glargine at a dose of 1.5 IU/animal. P values versus control are indicated by asterisks and p values of the low-dose insulin glargine versus the combination or the high-dose insuline glargine are indicated by crosses (one symbol, p < 0.05; two symbols, p < 0.01; three symbols, p < 0.001). Six hours after administration, the low-dose insuline glargine had reduced blood glucose by 22% versus control without showing statistically significant difference. The combination had decreased blood glucose by 49%, which was in the range of the high-dose insulin glargine with a decrease of 44%. Both treatments were significantly different from the control. The decreased blood glucose in the combination group did not show a statistically significant difference versus the high-dose insulin glargine group.

Reference **Example 3:**

The following example shows the beneficial effect on glycemic control of a SGLT2 inhibitor (compound (I.9)) sequentially added to an insulin (insulin glargine) as compared to the insulin alone. All experimental protocols concerning the use of laboratory animals were reviewed by a federal Ethics Committee and approved by governmental authorities. Two weeks before the study starts, the rats were pretreated with a single dose of 60 mg/kg i.p. of streptozotocin to induced experimental diabetes, resembling a type 1 diabetic condition. During the study blood glucose was followed over 8 h in male, non-fasted Sprague-Dawley rats (Crl:CD) with an age of 8-9 weeks at the start of the study. A pre-dose blood sample was obtained by tail bleed and blood glucose was measured with a glucometer 30, 60, 90 min and 2, 3, 4, 5, 6, 8 h after administration of insulin. At time point 0 min, the animals were injected with insulin glargine (n=10) or isotonic NaCl subcutaneously (n=5). At time point 120 (minutes) the insulin glargine-treated mice were randomized according to blood glucose and separated in 2 groups (n=5 per group). Animals received oral administrations of either vehicle alone (0.5% aqueous hydroxyethylcellulose) or this vehicle containing the SGLT2 inhibitor. The data are presented as mean±S.E.M. Statistical comparison was conducted by repeated measures two-way ANOVA followed by Bonferroni post tests for group-wise comparisons. A p value < 0.05 was considered to show a statistically significant difference. The result is shown in Figure 3. Insulin glargine was administered at a dose of 1.5 IU/animal. The term "Insuline glargine/ Cpd. A" denotes the combination of insulin glargine at a dose of 1.5 IU/animal and the SGLT2 inhibitor at a dose of 10 mg/kg. P values versus control are indicated by asterisks and p values of the vehicle-treated group versus the animals treated with the SGLT2 inhibitor are indicated by crosses (one symbol, p < 0.05; two symbols, p < 0.01; three symbols, p < 0.001). The blood glucose between 2 and 8 hours was significantly decreased by 50% when compared to the vehicle-treated mice.

Reference **Example 4:**

The following example shows the beneficial effect on body fat portion of a SGLT2 inhibitor (compound (I.9)) in combination with an insulin (as an insulin-releasing implant) as compared to the insulin (as an insulin-releasing implant) alone. All experimental protocols concerning the use of laboratory animals were reviewed by a federal Ethics Committee and approved by governmental authorities. Two weeks before the study starts, Sprague-Dawley rats (Crl:CD) were pretreated with a single dose of 60 mg/kg i.p. of streptozotocin to induced experimental diabetes, resembling a type 1 diabetic condition. Twice a day animals received oral administrations of either vehicle alone (0.5% aqueous hydroxyethylcellulose) or this vehicle containing the SGLT2 inhibitior (10 mg/kg). In additional groups, 1 or 2 insulin-releasing sticks were implanted subcutaneously in the neck of the rats. The SGLT2 inhibitor was administered to animals without or with 1 insulin implant. On day 27, the body fat was measured using the NMR technique. The data are presented as mean ±S.E.M. Statistical comparison was conducted by one-way ANOVA followed by Bonferroni post tests/unpaired t-test for group-wise comparisons. A p value < 0.05 was considered to show a statistically significant difference. The result is shown in Figure 4. The term "Cpd. A" denotes the SGLT2 inhibitor at a dose of 10 mg/kg. P values versus control are indicated by asterisks and p values of the animals receiving 1 implant versus the combination of 1 implant and the SGLT2 inhibitor

(denoted as "Cpd. A + 1 Implant") are indicated by crosses (one symbol, $p < 0.05$; two symbols, $p < 0.01$; three symbols, $p < 0.001$). Insulin-releasing implants significantly increased body fat portion (1 implant: +83%; 2 implants: +72%) when compared to controls.

The combination of 1 implant and the SGLT2 inhibitor (denoted as "Cpd. A + 1 Implant") showed significantly lower body fat when compared to the rats receiving 1 implant alone.

**Example 5:**

Treating patients with type 1 diabetes with the pharmaceutical composition according to the invention, in addition to producing an acute improvement in the glucose metabolic situation, may contribute to a sustainable well metabolic situation in the long term. This may be observed in patients being treated for a longer period, e.g. 3 months to 1 year or even 1 to 6 years, with the pharmaceutical composition according to the invention and compared with patients who are treated with insulin alone. There is evidence of therapeutic success compared with patients treated with insulin alone if no increase in the fasting glucose and/or HbA1c value is observed but where a reduction in hypoglycaemia event rate, glucose excursions or insulin requirement is seen. Further evidence of therapeutic success is obtained if a significantly smaller percentage of the patients treated with a pharmaceutical composition according to the invention, compared with patients who are treated with optimized insulin alone, undergo a deterioration in the glucose metabolic position (e.g. an increase in the HbA1c value to >6.5% or >7%). For example a clinical study with 30 patients with type 1 diabetes (for example utilizing insulin pumps or receiving a therapy comprising a long-acting insulin) can explore the effect of the SGLT2 inhibitor (in particular the compound (I.9), for example 10 or 25 mg once daily) as an adjunct to insulin with regards to safety and efficacy.

**Claims**

1. The SGLT2 inhibitor 1-chloro-4-(β-D-glucopyranos-1-yl)-2-[4-(*(S)*-tetrahydrofuran-3-yloxy)-benzyl]-benzene for use in a method for treating, preventing or reducing the risk of hypoglycemia in a patient in need thereof **characterized in that** the SGLT2 inhibitor is administered with an insulin.

2. The SGLT2 inhibitor according to claim 1 for use according to claim 1 **characterized in that** the SGLT2 inhibitor and the insulin are administered in combination or alternation to the patient.

3. The SGLT2 inhibitor according to claim 1 for use according to claim 1 or 2 **characterized in that** the patient shows one, two or more of the following conditions:

   (a) type 1 diabetes mellitus;
   (b) need for treatment with insulin;
   (c) latent autoimmune diabetes with onset in adults (LADA).

4. The SGLT2 inhibitor according to claim 1 for use according to one or more of the claims 1 to 3 **characterized in that** the patient is a patient who is or shall be treated with a insulin and shows one, two or more of the following conditions, including the risk to develop such conditions:

   (d) nocturnal and/or early morning hypoglycemia;
   (e) hypoglycemic episodes;
   (f) hyperglycemic episodes;
   (g) cardiac or cerebral complications;
   (h) retinopathy, in particular proliferative retinopathy;
   (i) injection site reactions, for example skin or subcutaneous tissue disorders.

5. The SGLT2 inhibitor according to claim 1 for use according to one or more of the claims 1 to 4 wherein the patient is an individual diagnosed of one or more of the conditions selected from the group consisting of overweight, obesity, visceral obesity and abdominal obesity and/or wherein the patient is a patient in which a weight increase is contraindicated.

6. The SGLT2 inhibitor according to claim 1 for use according to one or more of the claims 1 to 5 **characterized in that** the amount of the SGLT2 inhibitor is 1 to 25 mg per day.

7. The SGLT2 inhibitor according to claim 1 for use according to one or more of the claim 1 to 6 **characterized in that** the patient is a patient for which a reduction of the dose of the insulin is recommended.

8. The SGLT2 inhibitor according to claim 1 for use according to one or more of the claims 1 to 7 **characterized in that** the dose of the insulin is reduced compared with a monotherapy of said insulin.

9. The SGLT2 inhibitor according to claim 1 for use according to one or more of the claims 1 to 8 **characterized in that** the patient has insufficient glycemic control in particular despite treatment with an insulin, for example despite maximal recommended or tolerated dose of monotherapy with the insulin.

10. The SGLT2 inhibitor according to claim 1 for use according to one or more of the claims 1 to 9 wherein the insulin is selected from the group consisting of normal insulin, human insulin, zinc insulins and insulin analogues.

11. The SGLT2 inhibitor according to claim 1 for use according to one or more of the claims 1 to 10 wherein the insulin is selected from the group consisting of:

- rapid-acting insulins,
- short-acting insulins,
- intermediate-acting insulins,
- long-acting insulins,

including mixtures thereof.

12. The SGLT2 inhibitor according to claim 1 for use according to one or more of the claims 1 to 11 wherein the insulin is selected from the group consisting of insulin glargine, insulin detemir, insulin degludec, insulin lispro PEGylated and amidated insulin glargine.

13. The SGLT2 inhibitor according to claim 1 for use according to one or more of the claims 1 to 12 wherein the insulin is part of a basal insulin therapy.

14. The SGLT2 inhibitor according to claim 1 for use according to one or more of the claims 1 to 13 **characterized in that** the method is a first line, second line or third line therapy, or initial or add-on combination therapy or replacement therapy.

15. The SGLT2 inhibitor according to claim 1 for use according to one or more of the claims 1 to 14 comprising a further antidiabetic agent selected from the group consisting of biguanides, thiazolidindiones, sulfonylureas, glinides, inhibitors of alpha-glucosidase, GLP-1 analogues, DPP-4 inhibitors, for example linagliptin, and amylin analogs, including pharmaceutically acceptable salts of the beforementioned agents.

16. The SGLT2 inhibitor according to claim 1 for use according to one or more of the claims 1 to 15 wherein a pharmaceutical composition comprises the SGLT2 inhibitor and the insulin.

17. The SGLT2 inhibitor according to claim 1 for use according to claim 16 wherein the pharmaceutical composition is suitable for combined or simultaneous or sequential use of the SGLT2 inhibitor and the insulin.

## Patentansprüche

1. SGLT2-Inhibitor 1-Chlor-4-(β-D-glucopyranos-1-yl)-2-[4-(*(S)*-tetrahydrofuran-3-yloxy)-benzyl]-benzol zur Verwendung in einem Verfahren zur Behandlung, Vorbeugung oder Verringerung des Risikos einer Hypoglykämie bei einem Patienten mit Bedarf hierfür, **dadurch gekennzeichnet, dass** der SGLT2-Inhibitor mit einem Insulin verabreicht wird.

2. SGLT2-Inhibitor nach Anspruch 1 zur Verwendung nach Anspruch 1, **dadurch gekennzeichnet, dass** der SGLT2-Inhibitor und das Insulin dem Patienten in Kombination oder abwechselnd verabreicht werden.

3. SGLT2-Inhibitor nach Anspruch 1 zur Verwendung nach Anspruch 1 oder 2, **dadurch gekennzeichnet, dass** der Patient einen, zwei oder mehrere der folgenden Zustände zeigt:

(a) Diabetes mellitus Typ 1;
(b) Notwendigkeit der Behandlung mit Insulin,
(c) latente Autoimmundiabetes mit Beginn im Erwachsenenalter (LADA).

4. SGLT2-Inhibitor nach Anspruch 1 zur Verwendung nach einem oder mehreren der Ansprüche 1 bis 3, **dadurch gekennzeichnet, dass** der Patient ein Patient ist, der mit einem Insulin behandelt wird oder behandelt werden soll und einen, zwei oder mehrere der folgenden Zustände zeigt, einschließlich des Risikos, solche Zustände zu entwickeln:

(d) nächtliche und/oder frühmorgendliche Hypoglykämie;
(e) hypoglykämische Episoden;
(f) hyperglykämische Episoden;
(g) kardiale oder zerebrale Komplikationen;
(h) Retinopathie, insbesondere proliferative Retinopathie,
(i) Reaktionen an der Injektionsstelle, beispielsweise Störungen bzw. Erkrankungen der Haut oder des subkutanen Gewebes.

5. SGLT2-Inhibitor nach Anspruch 1 zur Verwendung nach einem oder mehreren der Ansprüche 1 bis 4, wobei es sich bei dem Patienten um ein Individuum handelt, bei dem eine oder mehrere der Erkrankungen diagnostiziert wurden, ausgewählt aus der Gruppe, bestehend aus Übergewicht, Adipositas, viszeraler Adipositas und abdominaler Adipositas, und/oder wobei es sich bei dem Patienten um einen Patienten handelt, bei dem eine Gewichtszunahme kontraindiziert ist.

6. SGLT2-Inhibitor nach Anspruch 1 zur Verwendung nach einem oder mehreren der Ansprüche 1 bis 5, **dadurch gekennzeichnet, dass** die Menge des SGLT2-Inhibitors 1 bis 25 mg pro Tag beträgt.

7. SGLT2-Inhibitor nach Anspruch 1 zur Verwendung nach einem oder mehreren der Ansprüche 1 bis 6, **dadurch gekennzeichnet, dass** der Patient ein Patient ist, für den eine Reduzierung der Insulindosis empfohlen wird.

8. SGLT2-Inhibitor nach Anspruch 1 zur Verwendung nach einem oder mehreren der Ansprüche 1 bis 7, **dadurch gekennzeichnet, dass** die Dosis des Insulins im Vergleich zu einer Monotherapie mit Insulin reduziert ist.

9. SGLT2-Inhibitor nach Anspruch 1 zur Verwendung nach einem oder mehreren der Ansprüche 1 bis 8, **dadurch gekennzeichnet, dass** der Patient eine unzureichende glykämische Kontrolle aufweist, insbesondere trotz Behandlung mit einem Insulin, beispielsweise trotz maximaler empfohlener oder tolerierter Dosis der Monotherapie mit dem Insulin.

10. SGLT2-Inhibitor nach Anspruch 1 zur Verwendung nach einem oder mehreren der Ansprüche 1 bis 9, wobei das Insulin ausgewählt ist aus der Gruppe, bestehend aus Normalinsulin, Humaninsulin, Zinkinsulinen und Insulinanaloga.

11. SGLT2-Inhibitor nach Anspruch 1 zur Verwendung nach einem oder mehreren der Ansprüche 1 bis 10, wobei das Insulin ausgewählt ist aus der Gruppe, bestehend aus:

- schnell wirkenden Insulinen,
- kurzwirkenden Insulinen,
- mittelschnellwirkenden Insulinen,
- langwirkenden Insulinen,

einschließlich Mischungen davon.

12. SGLT2-Inhibitor nach Anspruch 1 zur Verwendung nach einem oder mehreren der Ansprüche 1 bis 11, wobei das Insulin ausgewählt ist aus der Gruppen, bestehend aus Insulin glargin, Insulin detemir, Insulin degludec, PEGyliertem Insulin lispro und amidiertem Insulin glargin.

13. SGLT2-Inhibitor nach Anspruch 1 zur Verwendung nach einem oder mehreren der Ansprüche 1 bis 12, wobei das Insulin Teil einer Basalinsulintherapie ist.

**14.** SGLT2-Inhibitor nach Anspruch 1 zur Verwendung nach einem oder mehreren der Ansprüche 1 bis 13, **dadurch gekennzeichnet, dass** das Verfahren eine Erstlinien-, Zweitlinien- oder Drittlinientherapie oder eine anfängliche oder zusätzliche Kombinationstherapie oder Ersatztherapie ist.

**15.** SGLT2-Inhibitor nach Anspruch 1 zur Verwendung nach einem oder mehreren der Ansprüche 1 bis 14, umfassend ein weiteres antidiabetisches Mittel, ausgewählt aus der Gruppe bestehend aus Biguaniden, Thiazolidindionen, Sulfonylharnstoffen, Gliniden, Inhibitoren der alpha-Glucosidase, GLP-1-Analoga, DPP-4-Inhibitoren, beispielsweise Linagliptin, und Amylin-Analoga, einschließlich pharmazeutisch akzeptabler bzw. annehmbarer Salze der vorgenannten Mittel.

**16.** SGLT2-Inhibitor nach Anspruch 1 zur Verwendung nach einem oder mehreren der Ansprüche 1 bis 15, wobei eine pharmazeutische Zusammensetzung den SGLT2-Inhibitor und das Insulin umfasst.

**17.** SGLT2-Inhibitor nach Anspruch 1 zur Verwendung nach Anspruch 16, wobei die pharmazeutische Zusammensetzung zur kombinierten oder gleichzeitigen oder aufeinanderfolgenden Verwendung des SGLT2-Inhibitors und des Insulins geeignet ist.

## Revendications

**1.** Inhibiteur de SGLT2 1-chloro-4-(β-D-glucopyranos-1-yl)-2-[4-((*S*)-tétrahydrofuran-3-yloxy)-benzyl]-benzène pour son utilisation dans une méthode de traitement, de prévention ou de réduction du risque d'hypoglycémie chez un patient en ayant besoin, **caractérisé en ce que** l'inhibiteur de SGLT2 est administré avec une insuline.

**2.** Inhibiteur de SGLT2 selon la revendication 1 pour son utilisation selon la revendication 1, **caractérisé en ce que** l'inhibiteur de SGLT2 et l'insuline sont administrés en combinaison ou de manière alternée au patient.

**3.** Inhibiteur de SGLT2 selon la revendication 1 pour son utilisation selon la revendication 1 ou 2, **caractérisé en ce que** le patient présente une, deux ou plus de deux des conditions suivantes :

(a) diabète sucré de type 1 ;
(b) besoin d'un traitement à l'aide d'insuline ;
(c) diabète autoimmun latent avec apparition chez l'adulte (LADA).

**4.** Inhibiteur de SGLT2 selon la revendication 1 pour son utilisation selon une ou plusieurs des revendications 1 à 3, **caractérisé en ce que** le patient est un patient qui est ou doit être traité par une insuline et présente une, deux ou plus de deux des conditions suivantes, y compris le risque de développer de telles conditions :

(d) hypoglycémie nocturne et/ou tôt le matin ;
(e) épisodes hypoglycémiques ;
(f) épisodes hyperglycémiques ;
(g) complications cardiaques ou cérébrales ;
(h) rétinopathie, en particulier rétinopathie proliférative ;
(i) réactions au site d'injection, par exemple troubles de la peau ou du tissu sous-cutané.

**5.** Inhibiteur de SGLT2 selon la revendication 1 pour son utilisation selon une ou plusieurs des revendications 1 à 4, dans lequel le patient est un individu diagnostiqué comme ayant une ou plusieurs des conditions choisies dans le groupe constitué de surpoids, obésité, obésité viscérale et obésité abdominale et/ou dans lequel le patient est un patient chez qui une prise de poids est contre-indiquée.

**6.** Inhibiteur de SGLT2 selon la revendication 1 pour son utilisation selon une ou plusieurs des revendications 1 à 5, **caractérisé en ce que** la quantité de l'inhibiteur de SGLT2 est de 1 à 25 mg par jour.

**7.** Inhibiteur de SGLT2 selon la revendication 1 pour son utilisation selon une ou plusieurs des revendications 1 à 6, **caractérisé en ce que** le patient est un patient pour lequel une réduction de la dose de l'insuline est recommandée.

**8.** Inhibiteur de SGLT2 selon la revendication 1 pour son utilisation selon une ou plusieurs des revendications 1 à 7, **caractérisé en ce que** la dose de l'insuline est réduite par comparaison à une monothérapie de ladite insuline.

9. Inhibiteur de SGLT2 selon la revendication 1 pour son utilisation selon une ou plusieurs des revendications 1 à 8, **caractérisé en ce que** le patient présente une régulation glycémique insuffisante notamment malgré un traitement par une insuline, par exemple malgré une dose maximale recommandée ou tolérée de monothérapie par l'insuline.

10. Inhibiteur de SGLT2 selon la revendication 1 pour son utilisation selon une ou plusieurs des revendications 1 à 9, dans lequel l'insuline est choisie dans le groupe constitué d'insuline normale, insuline humaine, insulines de zinc et analogues d'insuline.

11. Inhibiteur de SGLT2 selon la revendication 1 pour son utilisation selon une ou plusieurs des revendications 1 à 10, dans lequel l'insuline est choisie dans le groupe constitué de :

    - insulines à action rapide,
    - insulines à courte durée d'action,
    - insulines à action intermédiaire,
    - insulines à longue durée d'action,

    y compris des mélanges de celles-ci.

12. Inhibiteur de SGLT2 selon la revendication 1 pour son utilisation selon une ou plusieurs des revendications 1 à 11, dans lequel l'insuline est choisie dans le groupe constitué d'insuline glargine, insuline détémir, insuline dégludec, insuline lispro PEGylée et insuline glargine amidée.

13. Inhibiteur de SGLT2 selon la revendication 1 pour son utilisation selon une ou plusieurs des revendications 1 à 12, dans lequel l'insuline fait partie d'une insulinothérapie basale.

14. Inhibiteur de SGLT2 selon la revendication 1 pour son utilisation selon une ou plusieurs des revendications 1 à 13, **caractérisé en ce que** la méthode est une thérapie de première, deuxième ou troisième intention, ou une thérapie combinée initiale ou d'appoint ou une thérapie de remplacement.

15. Inhibiteur de SGLT2 selon la revendication 1 pour son utilisation selon une ou plusieurs des revendications 1 à 14, comprenant un autre agent antidiabétique choisi dans le groupe constitué de biguanides, thiazolidinediones, sulfonylurées, glinides, inhibiteurs d'alpha-glucosidase, analogues de GLP-1, inhibiteurs de DPP-4, par exemple linagliptine, et analogues d'amyline, y compris des sels pharmaceutiquement acceptables des agents mentionnés ci-dessus.

16. Inhibiteur de SGLT2 selon la revendication 1 pour son utilisation selon une ou plusieurs des revendications 1 à 15, dans lequel une composition pharmaceutique comprend l'inhibiteur de SGLT2 et l'insuline.

17. Inhibiteur de SGLT2 selon la revendication 1 pour son utilisation selon la revendication 16, dans lequel la composition pharmaceutique est adaptée à une utilisation combinée ou simultanée ou séquentielle de l'inhibiteur de SGLT2 et de l'insuline.

Figure 1a

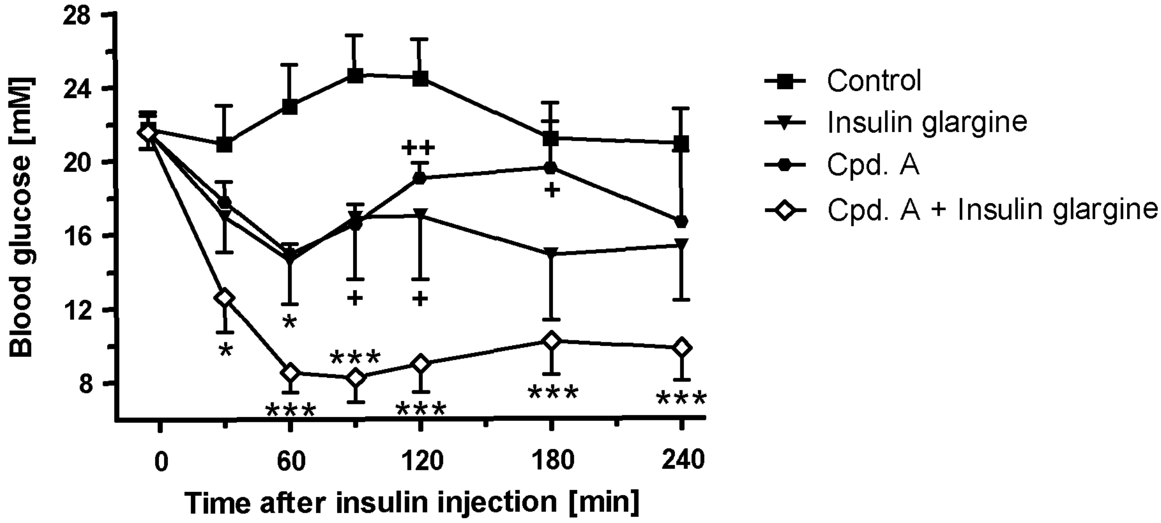


Figure 1b

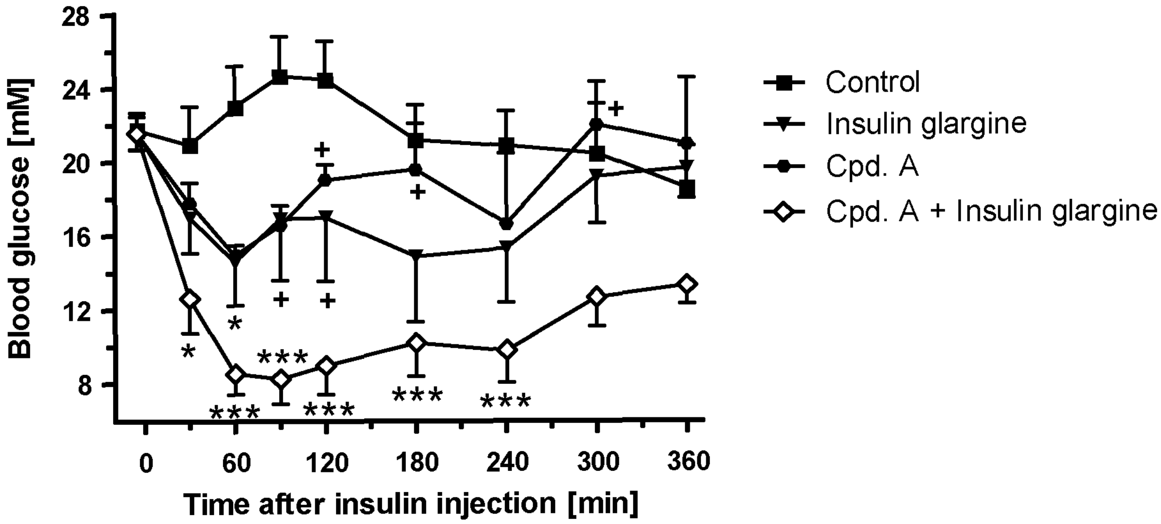

Figure 2a

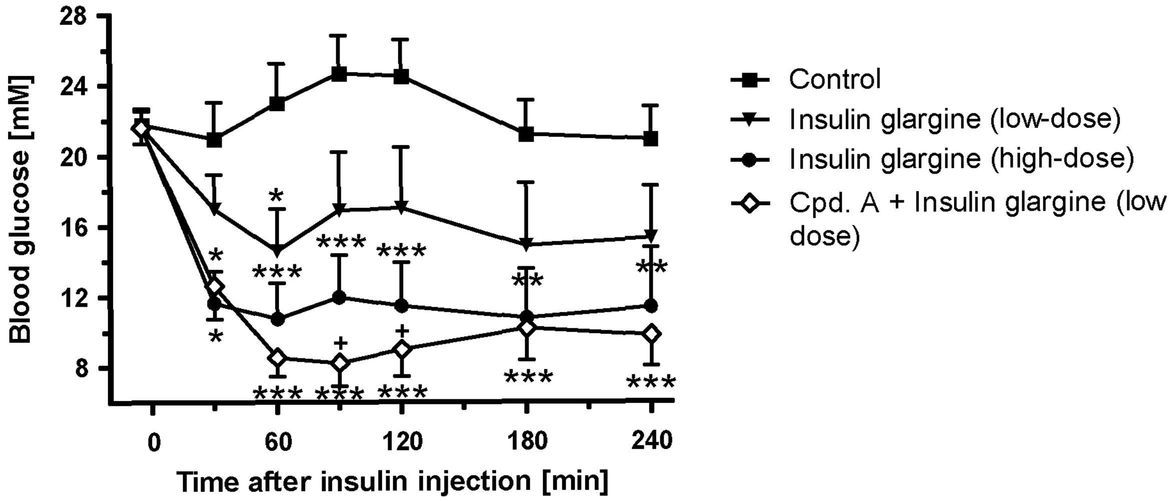


Figure 2b

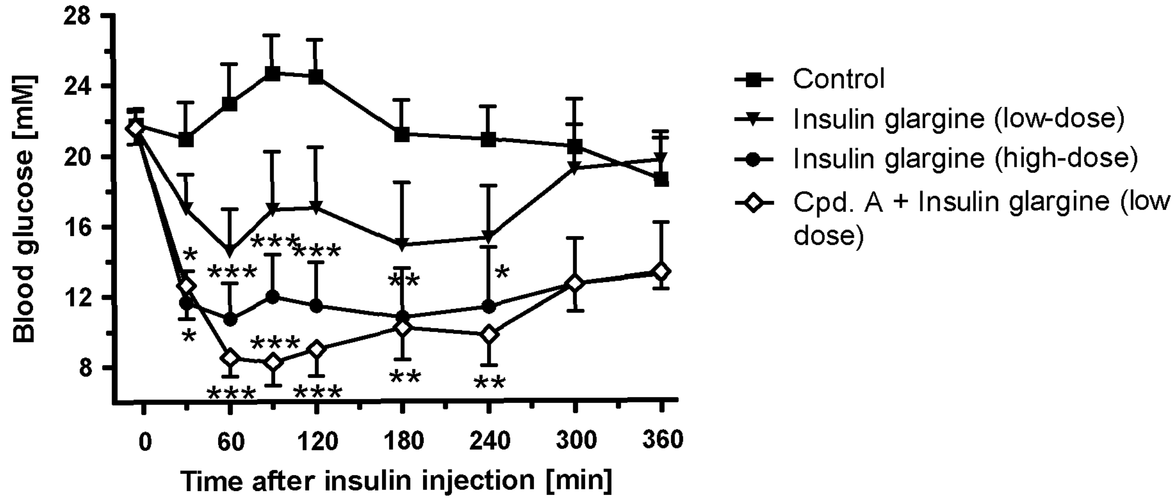

Figure 3

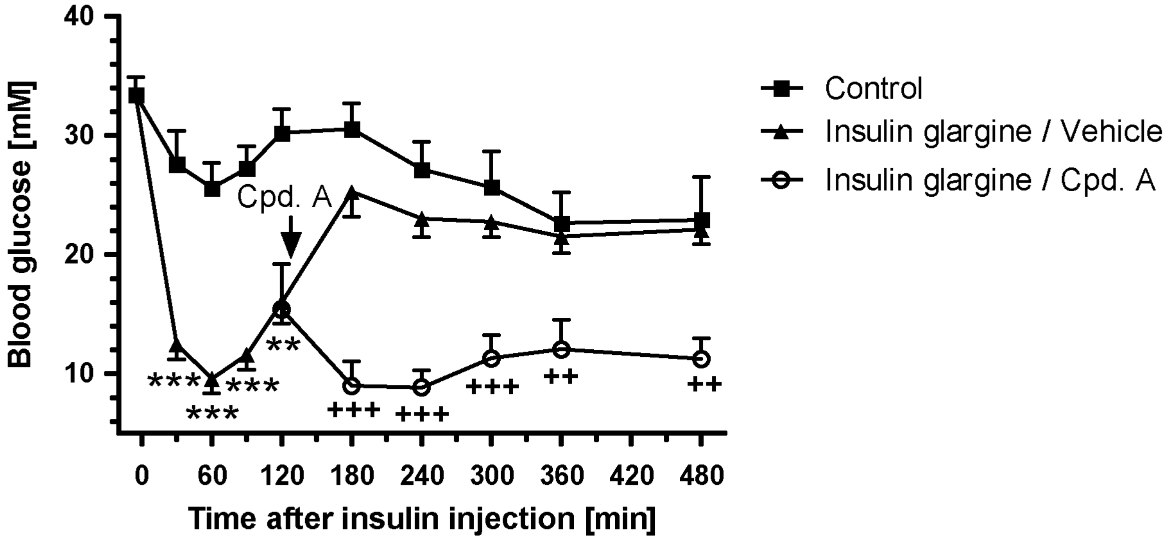


Figure 4

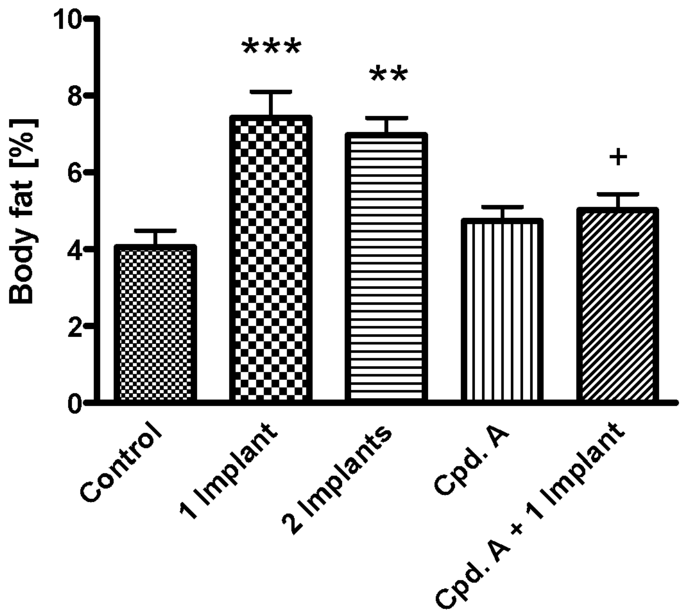

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0127128 A
- WO 03099836 A
- WO 2005092877 A
- WO 2006034489 A
- WO 2006064033 A
- WO 2006117359 A
- WO 2006117360 A
- WO 2007025943 A
- WO 2007028814 A
- WO 2007031548 A
- WO 2007093610 A
- WO 2007128749 A
- WO 2008049923 A
- WO 2008055870 A
- WO 2008055940 A
- WO 2006120208 A
- WO 2008020011 A
- WO 2009152128 A
- WO 2008006496 A
- WO 2009087082 A
- WO 2009087081 A
- WO 2010092126 A
- WO 2010092124 A


### Non-patent literature cited in the description


- **FORD ES et al.** *JAMA*, 2002, vol. 287, 356-9
- **KATSUKI A et al.** *Diabetes Care*, 2001, vol. 24, 362-5
- **MATTHEWS et al.** *, Diabetologia*, 1985, vol. 28, 412-19
- **FORST et al.** *, Diabetes*, 2003, vol. 52 (1), A459
- **GALVIN P et al.** *Diabet Med*, 1992, vol. 9, 921-8
- **MATTHEWS et al.** *Diabetologia*, 1985, vol. 28, 412-19
- **STUMVOLL et al.** *, Eur J Clin Invest*, 2001, vol. 31, 380-81
- **J. B. MEIGS et al.** *Diabetes*, 2003, vol. 52, 1475-1484
- *Diabetes Care*, 2002, vol. 25, 742-749
- **LAAKSONEN DE et al.** *Am J Epidemiol*, 2002, vol. 156, 1070-7
- *JAMA: Journal of the American Medical Association*, 2001, vol. 285, 2486-2497
- **LAAKSONEN DE et al.** *Am J Epidemiol.*, 2002, vol. 156, 1070-7